# EUROPEAN PATENT APPLICATION

(11) **EP 4 578 874 A1**
(43) Date of publication of application: **02.07.2025**
(21) Application number: 23383408.4
(22) Date of filing: 29.12.2023
(51) Int. Cl.: C07K 16/28

(54) **ANTI-CCR9 ANTIBODY**

(71) Applicant: SunRock Biopharma S.L., 15707 Santiago de Compostela (A Coruña) (ES); TOHOKU UNIVERSITY, Sendai-shi Miyagi 980-8577 (JP)
(72) Inventor: The designation of the inventor has not yet been filed
(74) Representative: Mewburn Ellis LLP

(57) **Abstract**

The present invention provides an anti-CCR9 antibody or antigen binding fragment thereof comprising a variable light chain comprising SEQ ID NO:1 or a variant thereof having at least 95% sequence identity to SEQ ID NO:1, wherein the variable light chain comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid. Also provided is the anti-CCR9 antibody or antigen binding fragment thereof for use in the treatment or prevention of a disease, as well as methods of diagnosis and antibody drug conjugates.

## Description

### Field of the Invention

The present invention relates to anti-CCR9 antibodies or antigen binding fragments thereof and uses thereof.

### Background

Chemokines are a family of small structurally related proteins that bind to seven transmembrane G protein-coupled receptors, with predominantly chemotactic function, currently composed of 44 members in humans. Chemokines and their receptors have an essential role in organogenesis and lymphocyte trafficking, in both homeostatic and inflammatory conditions. Chemokines generate gradients, either soluble or immobilized, and are responsible for tasks as diverse as directing cell movement, stimulating cell growth, activation or differentiation, or even regulating organogenesis in mammals (Zlotnik and Yoshie 2000, Raman, Sobolik-Delmaire et al. 2011). In the immune system, chemokines play a key role in homeostasis and in both innate and acquired immunity, by controlling the trafficking and recruitment of leukocytes.

Chemokines exert their biological effects through their interaction with specific receptors present on the cell surface. Structurally, these receptors belong to the G-protein-coupled seven-transmembrane-domain receptor (GPCR) superfamily (Bachelerie, Ben-Baruch et al. 2014). This system is characterized by key molecules in the activation of different genes, giving rise to different cellular responses that include chemotaxis, cell survival and proliferation (DeVries, Kelvin et al. 2006). There is a strong association between aberrant tumor cell expression of chemokine receptors such as CXCR4 or CCR7 and cancer progression, organ-selective metastasis, and poor prognosis. At the same time, they have great potential for use in tumor-targeted therapy (Weitzenfeld and Ben-Baruch 2014) in which, cancer-related chemokines could promote tumor proliferation, angiogenesis, and chemoresistance (Lazennec and Richmond 2010, Mukaida and Baba 2012, Sarvaiya, Guo et al. 2013).

Human chemokine receptor CCR9 (GenBank accession number. U45982) is a member of this superfamily of receptors, identified by Zaballos et al. (Zaballos, Gutiérrez et al. 1999) (EMBL database accession number AJ132337) and Youn et al. (Youn, Kim et al. 1999). Under physiological conditions, the expression of CCR9 is highly restricted: it has been described in thymocytes (Zaballos, Gutiérrez et al. 1999, Carramolino, Zaballos et al. 2001), infiltrating immune cells in the small intestine (Kunkel, Campbell et al. 2000) and in small subpopulations of circulating memory T cells (Zabel, Agace et al. 1999) and plasmacytoid dendritic cells (Wendland, Czeloth et al. 2007).

Unlike other chemokine receptors, CCR9 has a single ligand called CCL25 (Chemokine Ligand 25 or TECK) secreted by epithelial and dendritic cells from the thymus and the small intestinal crypt epithelium and that, when binding to its receptor, it activates intracellular signaling pathways related to cell survival and mobility (Wurbel, Malissen et al. 2006). The CCR9-CCL25 interaction is a key regulator of thymocyte migration in thymus and of cell homing to the intestinal tract. Recent insights into the mechanisms of CCL25/CCR9 show that they are involved in tumor chemoresistance and metastasis (Tu, Xiao et al. 2016), and the potential application of CCR9 in targeted therapies.

Expression of CCR9 is reported on the majority of gut-homing immune cells, including CD4+ and CD8+ T cells, gamma-delta T cells, plasmacytoid dendritic cells (pDCs), neutrophils and intraepithelial lymphocytes (IELs) (Pathak and Lal 2020; Wu *et al,* 2021). In this regard, evidence supports the role of the CCR9+-effector T-cells as a driver of inflammatory bowel disease (IBD) as clinical studies illustrate increased numbers of CCR9+ T cells in blood from individuals with small bowel inflammation (Trivedi and Adams, 2018) In addition to circulating gut homing T cells, subsets of proinflammatory monocytes also express high levels of CCR9 and their frequencies in the circulation correlate with clinical activity in some IBD patients (Trivedi and Adams, 2018).

Elevated CCR9 expression has been observed in various types of cancer. These include hematological tumors (T-cell acute lymphoblastic leukemia (T-ALL), follicular lymphoma and diffuse cell lymphoma B) (Qiuping, Z. et al. 2003, Wu, W. et al. 2014) and in solid tumors (lung, prostate, pancreas, breast and melanoma cancer). Such elevated expression of CCR9 would be advantageous for cells since, by binding to its ligand, CCL25, different signaling pathways would be activated, including PI3K/Akt, increasing the cell survival of transformed T lymphocytes and resistance to apoptosis mediated by this signaling pathway in different types of cancer (Sharma, Singh et al. 2010, Johnson-Holiday, Singh et al. 2011), as well as activation of the JNK1 anti-apoptotic pathway, and enhancement of proliferation by activating Notch1 in leukemia cells, especially in T-lineage acute lymphoblastic leukemia (T-ALL) (Mirandola, Chiriva-Internati et al. 2012). In addition, moderate levels of CCR9 expression have been observed in T cell chronic lymphocytic leukemia (T-CLL) CD4+ cells. In particular, when CCR9 is internalized into T-ALL CD4+ T cells, the chemotactic and adhesive ability of the leukemia cells are eliminated, indicating that CCR9 is closely associated with the infiltration and metastasis of leukemia cells.

Specific therapeutic tools to treat human CCR9⁺ tumours growing in xenogeneic models are limited to the use of toxin-coupled ligands (CCL25-PE38 fusion protein) (Hu et al., 2011, Leukaemia Res 35:1254-60) or ligand-specific antibodies alone or combined with the cytotoxic agent etoposide (Sharma et al., 2010, Int J Cancer 127:2020-30). In these strategies, the CCL25-CCR9 interaction is targeted to eliminate tumour cells; although the results have been limited, they provide evidence that CCR9 is a potential target for cancer immunotherapy.

Given the lack of therapies targeted to CCR9, there is still a need in the art to provide agents recognising CCR9 specifically that are suitable for the diagnosis, prognosis and/or treatment of a disease or condition concomitant with cells expressing CCR9.

WO2015/075269A1 discloses anti-CCR9 antibodies and their use in the treatment of cancers, including T cell acute lymphoblastic leukaemia (T-ALL), prostate cancer, breast cancer, melanoma, ovarian cancer, colorectal cancer and lung cancer. WO2015/075269A1 also discloses immunoconjugates in which the anti-CCR9 antibody is conjugated to or in the form of a fusion protein with another therapeutic agent such as a chemotherapeutic agent, including vincristine.

Despite the advances described above, there remains an urgent need for therapeutic strategies for the treatment of diseases such as inflammatory bowel disease and cancer, including T-cell acute lymphoblastic leukaemia (T-ALL) and pancreatic cancer. The present invention seeks to address this need and, as described in detail herein, provides further related advantages.

### Summary of the Invention

The present invention relates generally to anti-CCR9 antibodies or antigen binding fragments thereof.

According to a first aspect, there is provided an anti-CCR9 antibody or antigen binding fragment thereof, comprising a variable light chain comprising SEQ ID NO:1 or a variant thereof having at least 95% sequence identity to SEQ ID NO:1, wherein the variable light chain comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In some embodiments, the CDR-L1 comprises the amino acid sequence RSSQSLVHSNGITFLH (SEQ ID NO: 5) or the amino acid sequence RSSQSLLHSNGNTFLH (SEQ ID NO: 6). In some embodiments, the CDR-L3 comprises the amino acid sequence SQTTHVPWT (SEQ ID NO: 7) or the amino acid sequence SQTTHLPWT (SEQ ID NO: 8).

The antibody or antigen binding fragment thereof may comprise a CDR-H1 comprising the amino acid sequence GFTFSXYWMX (SEQ ID NO: 9), a CDR-H2 comprising the amino acid sequence EIRSKSXNYATHYXESXXG (SEQ ID NO: 10) and a CDR-H3 comprising the amino acid sequence XYSPY (SEQ ID NO: 11), wherein X can be any amino acid. In some embodiments, the CDR-H1 comprises the amino acid sequence GFTFSDYWMN (SEQ ID NO: 12) or the amino acid sequence GFTFSNYWMD (SEQ ID NO: 13). In some embodiments, the CDR-H2 comprises the amino acid sequence EIRSKSKNYATHYEESVRG (SEQ ID NO: 14) or the amino acid sequence EIRSKSNNYATHYAESMKG (SEQ ID NO: 15). The CDR-H3 may comprise the amino acid sequence FYSPY (SEQ ID NO: 16) or the amino acid sequence YYSPY (SEQ ID NO: 17).

In some embodiments, the antibody or antigen binding fragment thereof comprises a variable heavy chain comprising SEQ ID NO: 18 or a variant thereof having at least 95% sequence identity to SEQ ID NO: 18.

In some embodiments, the antibody or antigen binding fragment thereof comprises a variable heavy chain comprising SEQ ID NO: 18 or SEQ ID NO: 19. In some embodiments, the variable light chain comprises SEQ ID NO: 1 or SEQ ID NO: 20.

In some embodiments, the antibody or antigen binding fragment thereof comprises:
a variable light chain comprising SEQ ID NO:1, wherein the variable light chain comprises a CDR-L1 comprising the amino acid sequence RSSQSLVHSNGITFLH (SEQ ID NO: 5), a CDR-L2 comprising the amino acid sequence KVSNRFS (SEQ ID NO: 3), a CDR-L3 comprising the amino acid sequence SQTTHVPWT (SEQ ID NO: 7); and
a variable heavy chain comprising a CDR-H1 comprising the amino acid sequence GFTFSDYWMN (SEQ ID NO: 12), a CDR-H2 comprising the amino acid sequence EIRSKSKNYATHYEESVRG (SEQ ID NO: 14) and a CDR-H3 comprising the amino acid sequence FYSPY (SEQ ID NO: 16).

The antibody or antigen binding fragment thereof may bind to an epitope comprising amino acids 11, 12, 13, 15 and 16 of the N-terminal extracellular domain of CCR9 isoform A (SEQ ID NO: 21).

In some embodiments, the anti-CCR9 antibody or antigen binding fragment thereof comprises an IgG anti-CCR9 antibody.

In some embodiments, the antibody or antigen binding fragment exhibits a binding affinity dissociation constant K_{D} for human CCR9 of about 1 × 10⁻⁶ M or less.

Also provided by the present invention is an anti-CCR9 antibody or antigen binding fragment thereof comprising a variable light chain comprising SEQ ID NO:39, wherein SEQ ID NO: 39 comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In some embodiments, the CDR-L1 comprises the amino acid sequence RSSQSLVHSNGITFLH (SEQ ID NO: 5) and the CDR-L3 comprises the amino acid sequence SQTTHVPWT (SEQ ID NO: 7).

The present invention also provides an anti-CCR9 antibody or antigen binding fragment thereof comprising a variable light chain comprising SEQ ID NO: 40, wherein SEQ ID NO: 40 comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In some embodiments, the CDR-L1 comprises the amino acid sequence RSSQSLLHSNGNTFLH (SEQ ID NO: 6) and the CDR-L3 comprises the amino acid sequence SQTTHLPWT (SEQ ID NO: 8).

According to a further aspect of the invention, there is provided an anti-CCR9 antibody or antigen binding fragment thereof, wherein the anti-CCR9 antibody or antigen binding fragment thereof binds to a discontinuous epitope comprising amino acids at positions 11, 12, 13, 15 and 16 of the of the N-terminal extracellular domain of CCR9 isoform A (SEQ ID NO: 21). In some embodiments, the discontinuous epitope consists of amino acids at positions 11, 12, 13, 15 and 16 of the N-terminal extracellular domain of CCR9 isoform A (SEQ ID NO: 21). The anti-CCR9 antibody or antigen fragment thereof may be as defined in accordance with the first aspect of the invention.

Also provided is an antibody drug conjugate (ADC) comprising the anti-CCR9 antibody or antigen binding fragment thereof of any of the above aspects conjugated to a drug, optionally a cytotoxic or chemotherapy drug.

According to a further aspect, there is provided a polynucleotide comprising a nucleotide sequence encoding the anti-CCR9 antibody or antigen binding fragment thereof of any of the above aspects.

According to another aspect, there is provided a vector comprising the polynucleotide as described herein.

Also provided by the present invention is a cell comprising the anti-CCR9 antibody or antigen binding fragment thereof of the first aspect, the polynucleotide as described herein, or the vector as described herein.

According to a further aspect, there is provided a method of diagnosing a disease associated with elevated CCR9 expression in a subject, the method comprising:
a) contacting the antibody or antigen binding fragment thereof of the first aspect with a sample comprising cells from the subject;
b) detecting and/or quantifying CCR9 expression in the subject sample;
c) comparing the level of CCR9 expression detected in the subject sample with the level of CCR9 expression detected in a control sample;
d) correlating the comparison obtained in step c) with the presence of a disease associated with elevated CCR9 expression.

According to a further aspect, the present invention provides a pharmaceutical composition comprising the anti-CCR9 antibody or antigen binding fragment thereof of the first aspect, the ADC as described herein, the polynucleotide as described herein, the cell or the vector as described herein and a pharmaceutically or physiologically acceptable diluent and/or carrier.

There is also provided a kit comprising the anti-CCR9 antibody or antigen binding fragment thereof of the first aspect, the ADC as described herein, the polynucleotide as described herein, the vector as described herein, the cell as described herein or the pharmaceutical composition as described herein.

According to a further aspect of the invention, there is provided the anti-CCR9 antibody or antigen binding fragment thereof of the first aspect, the ADC, the polynucleotide as described herein, the vector as described herein, the cell or the pharmaceutical composition as described herein for use in the treatment or prevention of a disease. In some embodiments, the disease is an inflammatory disease. Preferably, the inflammatory disease is cancer or inflammatory bowel disease. In some embodiments the disease is inflammatory bowel disease. In some embodiments, the disease is selected from cancer, Crohn's disease, colitis, ulcerative colitis, inflammatory bowel disease, liver fibrosis and acute liver inflammation. The disease may be cancer. In some embodiments, the cancer is selected from ovarian cancer, prostate cancer, breast cancer, melanoma, T-cell acute lymphoblastic leukaemia (T-ALL), pancreatic cancer, colorectal cancer, lung cancer, circulating cells from a solid tumour (CTCs), T-cell lineage lymphomas, acute myeloid leukaemia (AML) and lung cancer.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

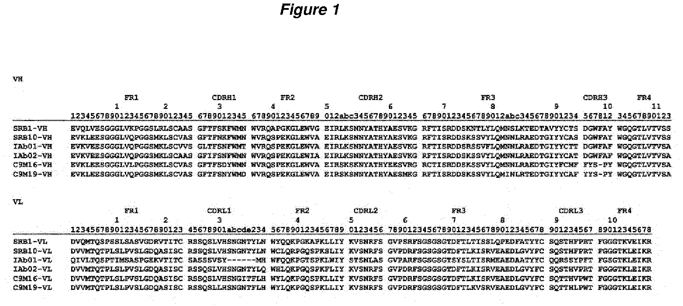

**Figure 1****. Alignment of the sequences of the variable domains of the anti-CCR9 antibodies.**

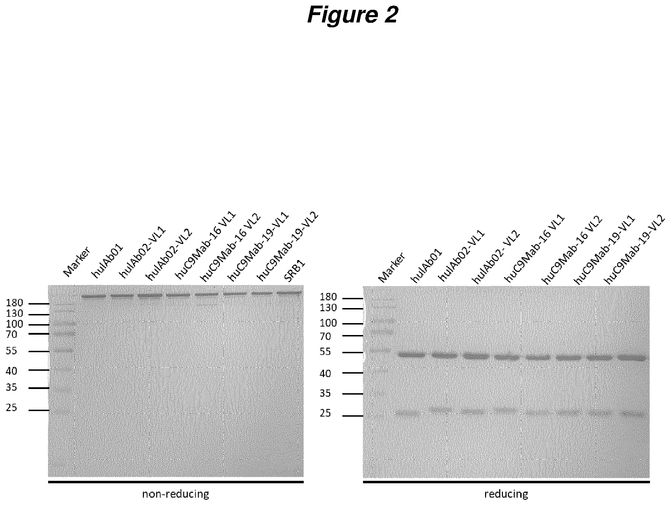

**Figure 2****. SDS-PAGE analysis of humanized anti-CCR9 antibodies.** SDS-PAGE analysis of the different anti-CCR9 antibodies (huIAb01, hulAb02-VL1, huIAb02-VL2, huC9Mab-16-VL1, huC9Mab-16-VL2, huC9Mab-19-VL1, and huC9Mab-19-VL2) under reducing and non-reducing conditions (12% PAA; 6 µg for reducing and 4 µg for non-reducing conditions).

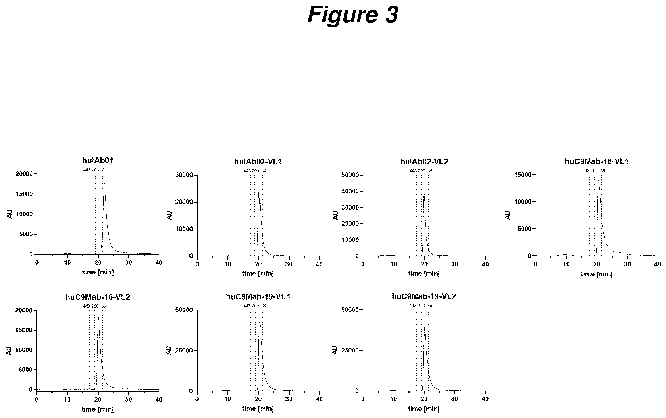

**Figure 3****. Size-exclusion chromatography of humanized anti-CCR9 antibodies.** Size-exclusion chromatography (SEC) of anti-CCR9 antibodies by HPLC using a TSKgelSuperSW mAb column.

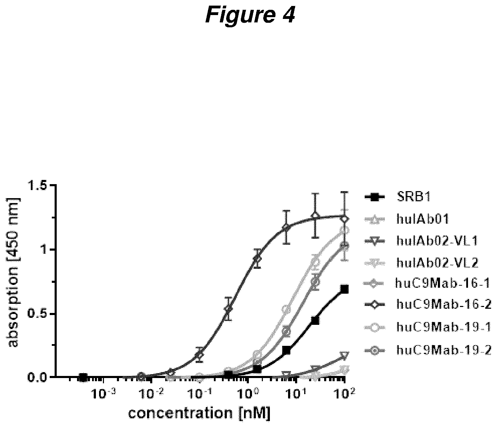

**Figure 4****. Binding of humanized antibodies.** Binding to the biotinylated peptide (TPTDFTSPIPNMADDYGSESTSS, SEQ ID NO: 22) from the N-terminal region of huCCR9 by ELISA. Mean ± SD; n=2.

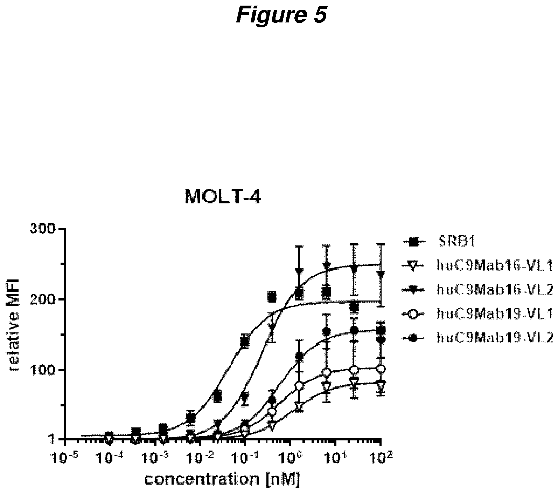

**Figure 5****. Binding analysis of humanized anti-CCR9 antibodies using MOLT-4 cells.** In this experiment, serial dilutions (1:4 titration) of anti-CCR9 antibodies were incubated for 1 hour at 4°C with cells. Bound antibodies were detected with a PE-labelled anti-human Fc antibody. Mean ± SD; n=3.

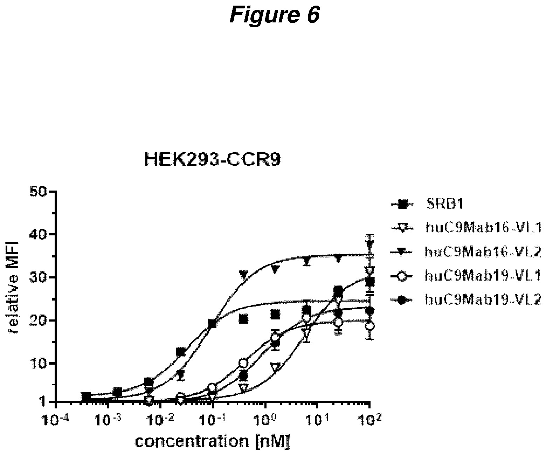

**Figure 6****. Binding analysis of humanized anti-CCR9 antibodies using HEK293-CCR9 cells.** In this experiment, serial dilutions (1:4 titration) of anti-CCR9 antibodies were incubated for 1 hour at 4°C with cells. Bound antibodies were detected with a PE-labelled anti-human Fc antibody. Mean ± SD; n=3.

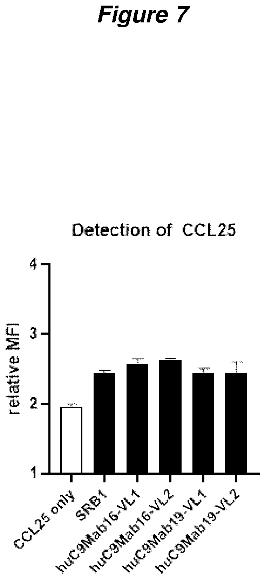

**Figure 7****. Blocking analysis of CCL25-moFc and humanized anti-CCR9 antibodies.** Detection of bound CCL25 to MOLT-4 cells alone and in the presence of CCR9 targeting antibodies. None of the candidates interfere with the binding of the ligand, suggesting different binding domains. In this experiment, 100 nM of humanized anti-CCR9 antibodies were incubated for 1 hour at 4°C with CCR9-expressing MOLT-4 cells. After three washing steps, 400 nM of CCL25 was added and incubated for 1 hour at 4°C. Bound ligand was detected with a FITC-labelled anti-moFc antibody. Mean ± SD; n=1.

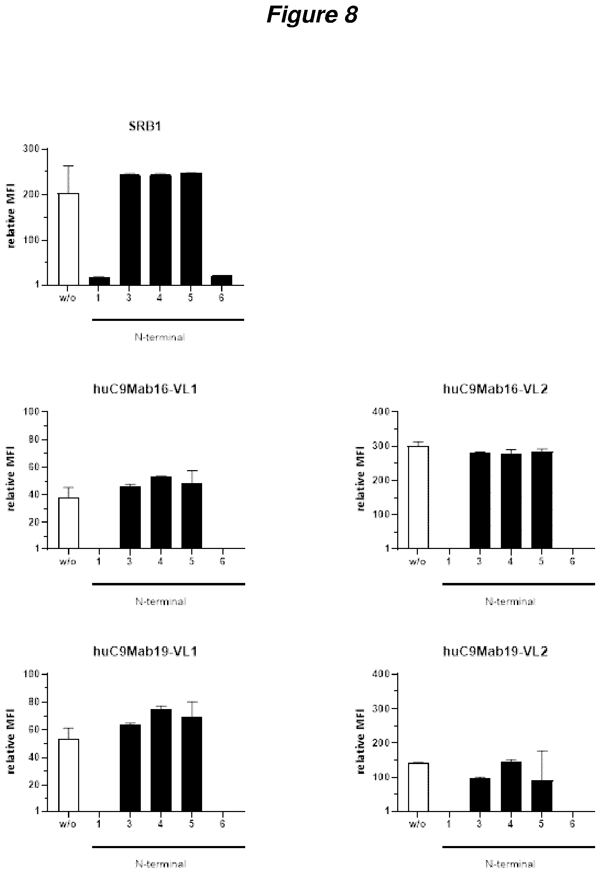

**Figure 8****. Peptide inhibition assay using MOLT-4 cells.** 1 µg/mL of antibodies was pre-incubated with 100 µg/mL of each peptide. After 1 hour at RT, the pre-incubation was transferred to MOLT-4 cells and incubated for 1 hour at 4 °C. Bound antibody was detected with a PE-labelled anti-human Fc antibody (Jackson ImmunoResearch 109-115-098; 1:500) and cells were analysed using a Miltenyi MACS-Quant. Mean ± SD; n=1.

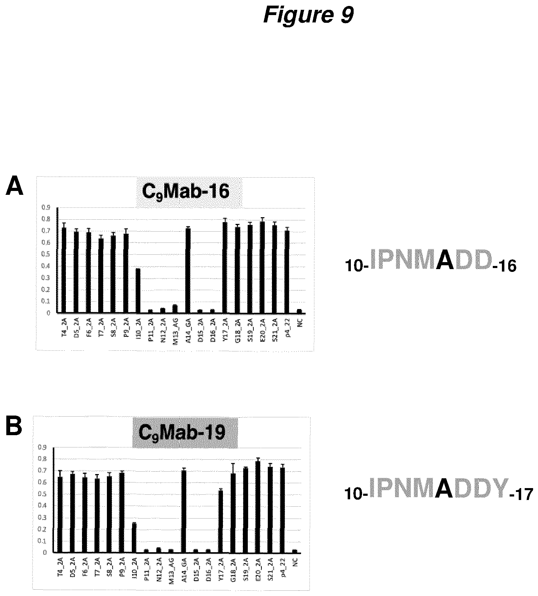

**Figure 9****. Epitope mapping of C9Mab-16 and C9Mab-19.** Binding analysis after 2 x Ala-scan method. The mutations showing reduced binding of the antibody candidates indicate a role of that residue on antibody binding. (A) C9Mab-16. (B) C9Mab-19.

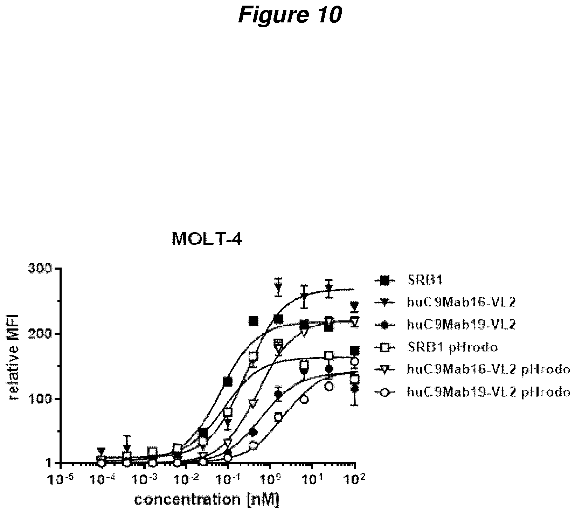

**Figure 10****. Binding analysis of pHrodo-labeled anti-CCR9 antibodies using MOLT-4 cells.** Flow cytometry analysis of pHrodo-labeled anti-CCR9 antibodies using CCR9-expressing MOLT-4 cells. In this experiment, serial dilutions (1:4 titration) of anti-CCR9 antibodies were incubated for 1 hour at 4°C with cells. Bound antibodies were detected with a PE-labelled anti-human Fc antibody. Mean ± SD; n=1.

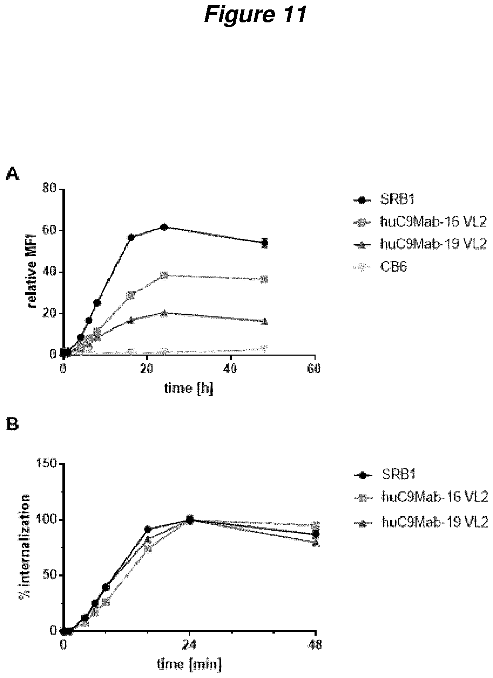

**Figure 11****. Internalization of pHrodo-labeled anti-CCR9 antibodies using MOLT-4 cells.** In this experiment 10 nM of pHrodo-labeled anti-CCR9 antibodies were incubated with cells for different incubation periods (5 minutes, 60 minutes, 4, hours, 6 hours, 8 hours, 16 hours, 24 hours, and 48 hours) at 37°C. Internalization was quantified as fluorescent signals were generated upon processing of the internalized antibody into the acidic endosome and lysosome pathway. CB6 is the receptor binding domain of the human anti-SARS-CoV-2 spike protein, employed as a control of unspecific internalization (A). Normalization of the internalization percentages of each molecule was performed to show the point at which their internalization is maximal (B). Mean ± SD; n=1.

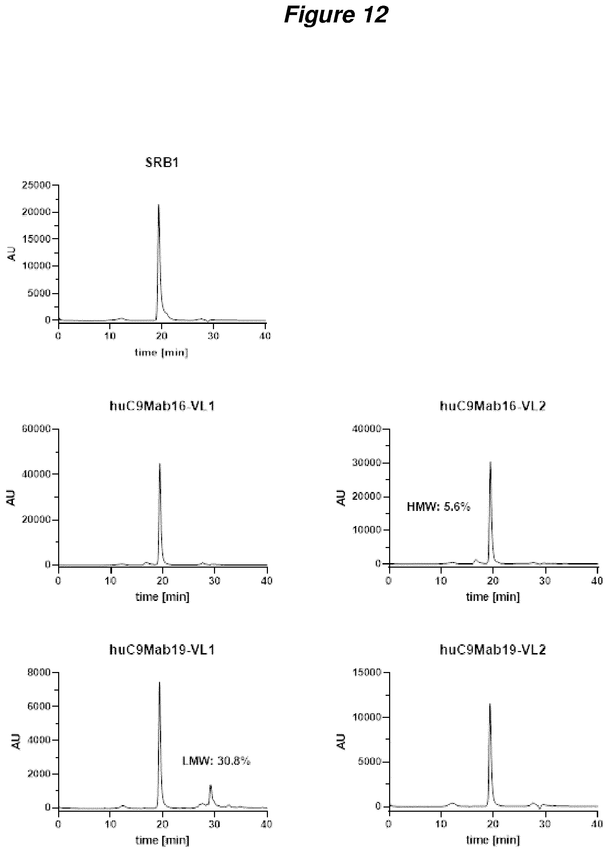

**Figure 12****. Size-exclusion chromatography of humanized anti-CCR9 antibodies after 4 freeze-thaw-cycles.** Anti-CCR9 antibodies were frozen at -80°C for up to one day and thawed on ice. The process was repeated for 3 times. After 4 days, the thawed antibodies were analysed using SEC-HPLC (TSKgelSuperSW mAb column).

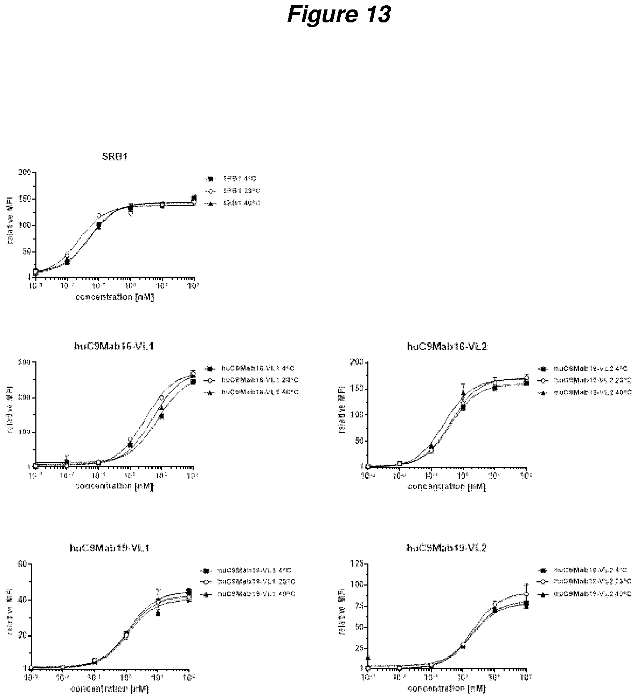

**Figure 13****. Binding analysis of anti-CCR9 antibodies under thermal stress using MOLT-4 cells.** Flow cytometry analysis of anti-CCR9 antibodies at 4°C, 23°C, and 40°C using CCR9-expressing MOLT-4 cells. Prior to flow cytometry analysis, the antibodies were incubated for 4 days at 4°C, 23°C, and 40°C. Then, serial dilutions (1:10 titration) of anti-CCR9 antibodies were incubated for 1 hour at 4°C with cells. Bound antibodies were detected with a PE-labelled anti-human Fc antibody. The measurement of huC9Mab16-VL2, huC9Mab19-VL1 and huC9Mab19-VL2 was interrupted, so that the measurement was incomplete. Mean ± SD; n=1.

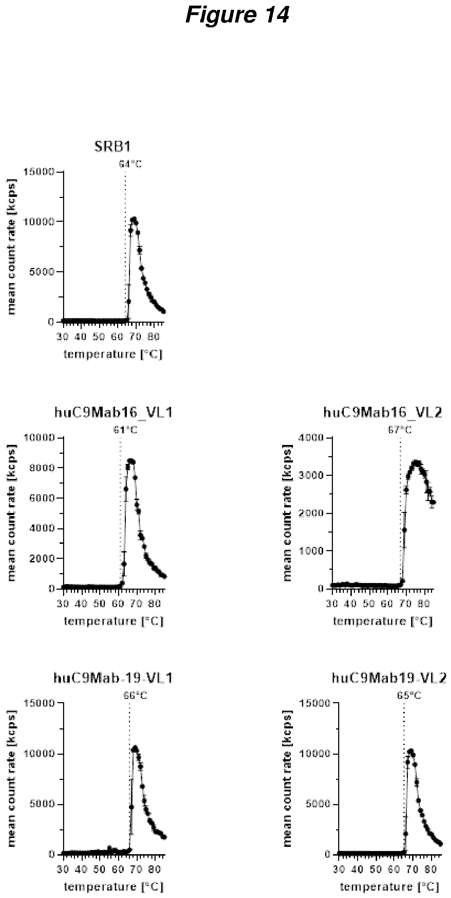

**Figure 14****. Dynamic light scattering of humanized anti-CCR9 antibodies.** Dynamic laser light scattering with ZetaSizer Nano ZS (Malvern) was measured while the temperature was increased in 1°C intervals from 35°C to 80°C with 2 minutes equilibration time for each temperature step. The aggregation point of the antibodies was defined as the temperature at which the mean count rate was increased. Mean ± SD; n=1

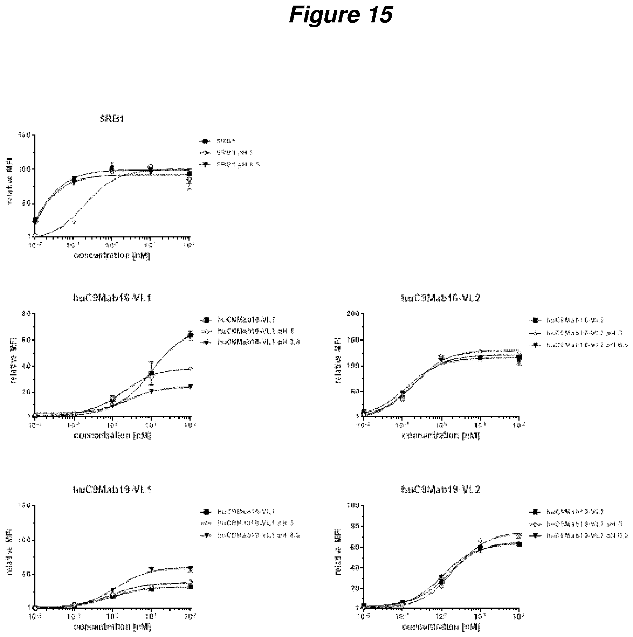

**Figure 15****. Binding analysis of anti-CCR9 antibodies under pH stress using MOLT-4 cells.** Flow cytometry analysis of anti-CCR9 antibodies at pH 5 and pH 8.5 using CCR9-expressing MOLT-4 cells. Prior to flow cytometry analysis, the antibodies were respectively dialyzed overnight in 100 mM sodium acetate (with 150 mM NaCl) at pH 5 and in 100 mM Tris-HCl (with 150 mM NaCl) at pH 8.5. Next day, concentration of the antibodies was measured, and the antibodies were incubated for 7 days at 40°C. Then, serial dilutions (1:10 titration) of anti-CCR9 antibodies were incubated for 1 hour at 4°C with cells. Bound antibodies were detected with a PE-labelled anti-human Fc antibody. Mean ± SD; n=1.

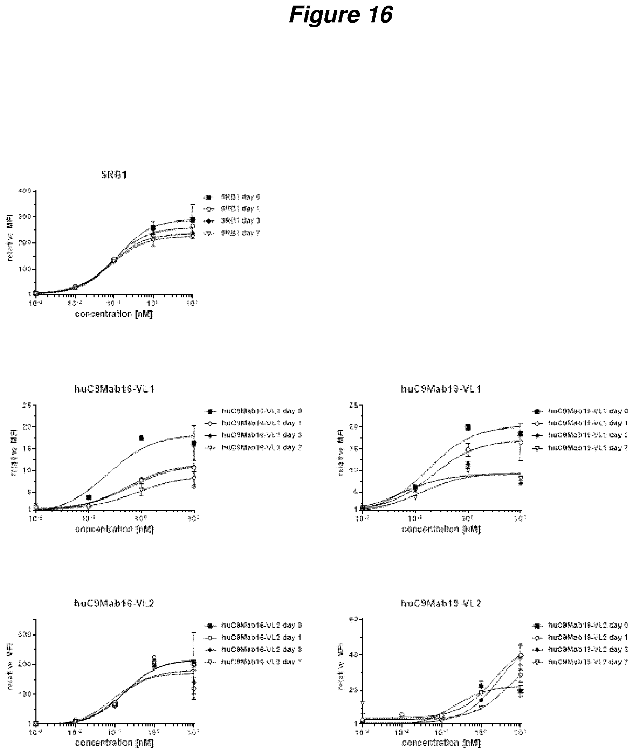

**Figure 16****. Binding analysis of anti-CCR9 antibodies, which were exposed to human serum using MOLT-4 cells.** Flow cytometry analysis of anti-CCR9 antibodies, which were incubated for 0, 1, 3, and 7 days to human serum using CCR9-expressing MOLT-4 cells. After the respective time of incubation, antibodies were stored at -20°C. In this experiment, serial dilutions (1:10 titration) of anti-CCR9 antibodies were incubated for 1 hour at 4°C with cells. Bound antibodies were detected with a PE-labelled anti-human Fc antibody. Mean ± SD; n=1.

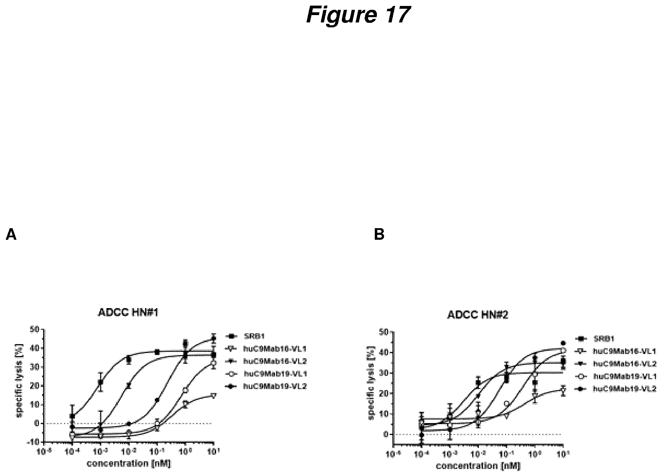

**Figure 17****. ADCC analysis of anti-CCR9 antibodies using CFSE-stained MOLT-4 cells as well as two different PBMC batches.** MOLT-4 cells were stained with CFSE, and then 2 × 104 cells were seeded overnight. The proportion of NK cells of two different PBMC batches were determined via flow cytometry analysis using anti-huCD3-PE and anti-huCD56-APC. PBMC batch HN#1 (A) displayed a proportion of NK cells of 12%, while PBMC batch HN#2 (B) exhibited 3.6%. CFSE-stained MOLT4 cells were pre-incubated with serial dilutions of the anti-CCR9 antibodies. Then, PBMCs were added on top to reach a concentration of 2 × 104 NK cells per well (equals target-to-effector ratio 1 :1). After incubating the plates for 24 hours, the cells were resuspended in PBA containing PI and the viability of the CFSE-labelled MOLT-4 cells was determined by flow cytometry. For representation of data as specific lysis, all values were normalized to the control wells containing CFSE-stained MOLT-4 and PBMCs. Mean ± SD; n=1.

### Detailed Description of the Invention

### CCR9

Chemokines are a family of small, structurally related proteins that bind to seven transmembrane spanning G protein-coupled receptors. Chemokines and their receptors have an essential role in organogenesis and lymphocyte trafficking, in both homeostatic and inflammatory conditions. The antibody or antigen binding fragment thereof of the invention binds specifically to CCR9.

The amino acid sequence of human CCR9 (C-C chemokine receptor type 9) is disclosed at the UniProt accession number **P51686** (see version 2 of 5 September 2006), the entire contents of which is incorporated herein by reference. Two alternatively spliced transcript variants have been described for the gene encoding CCR9, resulting in CCR9 isoform A and CCR9 isoform B, with UniProt accession numbers P51686-1 and P51686-2, respectively (see version 2 of 5 September 2006).

The term "CCR9" or "CCR9 chemokine receptor", as used herein, refers to the chemokine (C-C motif) receptor 9. Other terms to designate CCR9 include GPR28; CDw199; GPR-9-6; CC-CKR-9. A specific ligand of CCR9 is CCL25. Typically, the chemokine receptor CCR9 is expressed on most small intestinal lamina propria and intraepithelial lymphocytes and on a small subset of peripheral blood lymphocytes. Generally, CCR9 is expressed on the majority of thymocytes, small intestinal lamina propria and intraepithelial lymphocytes, on a small subset of peripheral blood lymphocytes and in a subset of memory T cells α₄β₇ present in the circulation. It can also be expressed on IgA secreting B cells, macrophages and plasmacytoid dendritic cells.

CCR9 is organized into 15 domains, corresponding to an N-terminal extracellular domain (Nt), seven transmembrane domains, three intracellular domains, three extracellular domains and an intracellular C-terminal domain (Ct). In some embodiments, the epitope comprises amino acids at positions 11, 12, 13, 15 and 16 of the N-terminal extracellular domain of CCR9 isoform A. SEQ ID NO: 21 represents amino acids at positions 2-22 of the N-terminal extracellular domain of CCR9 isoform A.

### Anti-CCR9 antibody or antigen binding fragment thereof

According to a first aspect, there is provided an anti-CCR9 antibody or antigen binding fragment thereof, comprising a variable light chain comprising SEQ ID NO:1 or a variant thereof having at least 95% sequence identity to SEQ ID NO:1, wherein the variable light chain comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In the context of the present invention, "anti-CCR9", will be understood to mean that the antibody or antigen binding fragment thereof specifically binds to CCR9. Specifically binds in this context may be distinguished from non-specific binding by virtue of the degree of binding affinity and/or the selectivity of binding, wherein the binding affinity for CCR9 is greater than for other antigens. In the context of the present invention, a greater binding affinity may refer to a lower K_{D} value.

The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue:W34-40), by following the numbering provided by Kabat et al., Sequences of Proteins of Immunological Interest, 5th ed.,Public Health Service, National Institutes of Health, Bethesda, Md. (1991), as is used in the present application, and/or by following the numbering provided by Chothia et al. (1989, Nature 342:877-83).

The antibody or antigen binding fragment thereof may be selected from the group consisting of: Fv, Fab, F(ab')₂, Fab', scFv, scFv-Fc, minibody, nanobody and diabody.

In some embodiments, the antigen binding fragment comprises an Fv, Fab, F(ab')₂, Fab', scFv, scFv-Fc, minibody or nanobody.

In some embodiments, the anti-CCR9 antibody or antigen binding fragment thereof comprises an anti-CCR9 antibody.

The term "variant" as used herein, will be understood to refer to a sequence which does not have 100% sequence identity to its reference sequence, for example, SEQ ID NO:1, but remains functionally active. For example, a variant of SEQ ID NO:1 of the invention will retain the functional activity of specifically binding to CCR9. The functional activity may be equivalent to the functional activity of the reference sequence, improved compared to the functional activity of the reference sequence or reduced compared to the functional activity of the reference sequence. In some embodiments, the variant has at least about 50% of the functional activity of the reference sequence. Optionally, the variant has at least about 60%, at least about 70%, at least about 80%, at least about 90% or at least about 95% of the functional activity of the reference sequence.

In the context of the present invention, a variant may comprise a sequence comprising one or more amino acid additions, substitutions or deletions relative to the reference sequence. For example, the variant may comprise a sequence comprising one or more substitutions relative to the reference sequence. In some embodiments, the variant comprises a sequence comprising at least two and optionally at least three amino acid substitutions relative to the reference sequence.

The variant may have a sequence identity of at least 96%, at least 97%, at least 98% or at least 99% to SEQ ID NO: 1. In some embodiments, the variant has a sequence identity of at least 98% to SEQ ID NO: 1.

In some embodiments, the variable light chain X amino acid is an aliphatic amino acid. As the skilled person will appreciate, aliphatic amino acids do not comprise cyclic structures with alternating double-bonds. Examples of aliphatic amino acids include, but are not necessarily limited to alanine, isoleucine, leucine, proline, valine and asparagine.

In some embodiments, the variable light chain X amino acid is an aliphatic non-polar amino acid or asparagine. In some embodiments, X is selected from the group consisting of valine, leucine, isoleucine and asparagine. In some embodiments, X is selected from the group consisting of valine, leucine and isoleucine.

In some embodiments, the CDR-L1 comprises or consists of the amino acid sequence RSSQSLVHSNGITFLH (SEQ ID NO: 5) or the amino acid sequence RSSQSLLHSNGNTFLH (SEQ ID NO: 6). In some embodiments, the CDR-L1 comprises or consists of the amino acid sequence RSSQSLVHSNGITFLH (SEQ ID NO: 5). Alternatively, the CDR-L1 may comprise or consist of the amino acid sequence RSSQSLLHSNGNTFLH (SEQ ID NO: 6).

In some embodiments, the CDR-L3 comprises or consists of the amino acid sequence SQTTHVPWT (SEQ ID NO: 7) or the amino acid sequence SQTTHLPWT (SEQ ID NO: 8). The CDR-L3 may comprise or consist of the amino acid sequence SQTTHVPWT (SEQ ID NO: 7). In some embodiments, the CDR-L3 may comprise or consist of the amino acid sequence SQTTHLPWT (SEQ ID NO: 8).

In some embodiments, the CDR-L1 comprises or consists of the amino acid sequence RSSQSLVHSNGITFLH (SEQ ID NO: 5), the CDR-L2 comprises or consists of the amino acid sequence KVSNRFS (SEQ ID NO: 3) and the CDR-L3 comprises or consists of the amino acid sequence SQTTHVPWT (SEQ ID NO: 7).

In some embodiments, the CDR-L1 comprises or consists of the amino acid sequence RSSQSLLHSNGNTFLH (SEQ ID NO: 6), the CDR-L2 comprises or consists of the amino acid sequence KVSNRFS (SEQ ID NO: 3) and the CDR-L3 comprises or consists of the amino acid sequence SQTTHLPWT (SEQ ID NO: 8).

The antibody or antigen binding fragment thereof may comprise a CDR-H1 comprising the amino acid sequence GFTFSXYWMX (SEQ ID NO: 9), a CDR-H2 comprising the amino acid sequence EIRSKSXNYATHYXESXXG (SEQ ID NO: 10) and a CDR-H3 comprising the amino acid sequence XYSPY (SEQ ID NO: 11), wherein X can be any amino acid.

In some embodiments, the variable heavy chain X amino acid is selected from the group consisting of asparagine, aspartic acid, lysine, glutamic acid, valine, arginine, alanine, methionine, phenylalanine and tyrosine.

In some embodiments, the CDR-H1 comprises or consists of the amino acid sequence GFTFSDYWMN (SEQ ID NO: 12) or the amino acid sequence GFTFSNYWMD (SEQ ID NO: 13). In some embodiments, the CDR-H1 comprises or consists of the amino acid sequence GFTFSDYWMN (SEQ ID NO: 12). In other embodiments, the CDR-H1 comprises or consists of the amino acid sequence GFTFSNYWMD (SEQ ID NO: 13).

In some embodiments, the CDR-H2 comprises or consists of the amino acid sequence EIRSKSKNYATHYEESVRG (SEQ ID NO: 14) or the amino acid sequence EIRSKSNNYATHYAESMKG (SEQ ID NO: 15). In some embodiments, the CDR-H2 comprises or consists of the amino acid sequence EIRSKSKNYATHYEESVRG (SEQ ID NO: 14). In other embodiments, the CDR-H2 comprises or consists of the amino acid sequence EIRSKSNNYATHYAESMKG (SEQ ID NO: 15).

The CDR-H3 may comprise or consist of the amino acid sequence FYSPY (SEQ ID NO: 16) or the amino acid sequence YYSPY (SEQ ID NO: 17). In some embodiments, the CDR-H3 comprises or consists of the amino acid sequence FYSPY (SEQ ID NO: 16). In some embodiments, the CDR-H3 comprises or consists of the amino acid sequence YYSPY (SEQ ID NO: 17).

In some embodiments, the CDR-H3 comprises the amino acid sequence XFXYSPY (SEQ ID NO: 82). For example, the CDR-H3 may comprise the amino acid sequence MFXYSPY (SEQ ID NO: 83) or AFXYSPY (SEQ ID NO: 84). In some embodiments, the CDR-H3 comprises the amino acid sequence MFFYSPY (SEQ ID NO: 85). In some embodiments, the CDR-H3 comprises the amino acid sequence AFYYSPY (SEQ ID NO: 86).

The antibody or antigen binding fragment thereof may comprise a variable heavy chain comprising a CDR-H1, CDR-H2 and CDR-H3 as defined above.

The antibody or antigen binding fragment thereof may comprise a variable heavy chain comprising a CDR-H1 comprising the amino acid sequence GFTFSDYWMN (SEQ ID NO: 12), a CDR-H2 comprising the amino acid sequence EIRSKSKNYATHYEESVRG (SEQ ID NO: 14) and a CDR-H3 comprising the amino acid sequence FYSPY (SEQ ID NO: 16).

Alternatively, the antibody or antigen binding fragment thereof may comprise a variable heavy chain comprising a CDR-H1 comprising the amino acid sequence GFTFSNYWMD (SEQ ID NO: 13), a CDR-H2 comprising the amino acid sequence EIRSKSNNYATHYAESMKG (SEQ ID NO: 15) and a CDR-H3 comprising the amino acid sequence YYSPY (SEQ ID NO: 17).

In some embodiments, the antibody or antigen binding fragment thereof comprises a variable heavy chain comprising SEQ ID NO: 18 or a variant thereof having at least 95% sequence identity to SEQ ID NO: 18. The variant may have a sequence identity of at least 96%, at least 97%, at least 98% or at least 99% to SEQ ID NO: 18. In some embodiments, the variant has a sequence identity of at least 97% to SEQ ID NO: 18.

In some embodiments, the antibody or antigen binding fragment thereof comprises a variable heavy chain comprising SEQ ID NO: 18 or a variant thereof having at least 95% sequence identity to SEQ ID NO: 18, wherein the heavy chain comprises a CDR-H1 comprising the amino acid sequence GFTFSXYWMX (SEQ ID NO: 9), a CDR-H2 comprising the amino acid sequence EIRSKSXNYATHYXESXXG (SEQ ID NO: 10) and a CDR-H3 comprising the amino acid sequence XYSPY (SEQ ID NO: 11), wherein X can be any amino acid.

In some embodiments, the antibody or antigen binding fragment thereof comprises a variable heavy chain comprising or consisting of SEQ ID NO: 18 or SEQ ID NO: 19. In some embodiments, the antibody or antigen binding fragment thereof comprises a variable heavy chain comprising or consisting of SEQ ID NO: 18. In some embodiments, the antibody or antigen binding fragment thereof comprises a variable heavy chain comprising or consisting of SEQ ID NO: 19.

In some embodiments, the variable light chain comprises or consists of SEQ ID NO: 1 or SEQ ID NO: 20. The variable light chain may comprise or consist of SEQ ID NO: 1. In other embodiments, the variable chain comprises or consists of SEQ ID NO: 20.

In some embodiments, the antibody or antigen binding fragment thereof comprises:
a. A variable light chain comprising SEQ ID NO:1, wherein the variable light chain comprises a CDR-L1 comprising the amino acid sequence RSSQSLVHSNGITFLH (SEQ ID NO: 5), a CDR-L2 comprising the amino acid sequence KVSNRFS (SEQ ID NO: 3), a CDR-L3 comprising the amino acid sequence SQTTHVPWT (SEQ ID NO: 7); and
b. A variable heavy chain comprising a CDR-H1 comprising the amino acid sequence GFTFSDYWMN (SEQ ID NO: 12), a CDR-H2 comprising the amino acid sequence EIRSKSKNYATHYEESVRG (SEQ ID NO: 14) and a CDR-H3 comprising the amino acid sequence FYSPY (SEQ ID NO: 16).

In some embodiments, the antibody or antigen binding fragment thereof comprises:
a. A variable light chain comprising SEQ ID NO: 20, wherein the variable light chain comprises a CDR-L1 comprises amino acid sequence RSSQSLLHSNGNTFLH (SEQ ID NO: 6), a CDR-L2 comprising the amino acid sequence KVSNRFS (SEQ ID NO: 3), a CDR-L3 comprising the amino acid sequence SQTTHLPWT (SEQ ID NO: 8); and
b. A variable heavy chain comprising a CDR-H1 comprising the amino acid sequence GFTFSNYWMD (SEQ ID NO: 13), a CDR-H2 comprising the amino acid sequence EIRSKSNNYATHYAESMKG (SEQ ID NO: 15) and a CDR-H3 comprising the amino acid sequence YYSPY (SEQ ID NO: 17).

In some embodiments, the antibody or antigen binding fragment thereof comprises:
a) a variable light chain comprising or consisting of SEQ ID NO: 1 and a variable heavy chain comprising or consisting of SEQ ID NO: 18; or
b) a variable light chain comprising or consisting of SEQ ID NO: 20 and a variable heavy chain comprising or consisting of SEQ ID NO: 19.

In some embodiments, the antibody or antigen binding fragment thereof comprises a variable light chain comprising or consisting of SEQ ID NO: 1 and a variable heavy chain comprising or consisting of SEQ ID NO: 18.

In some embodiments, the antibody or antigen binding fragment thereof comprises a variable light chain comprising or consisting of SEQ ID NO: 20 and a variable heavy chain comprising or consisting of SEQ ID NO: 19.

The antibody or antigen binding fragment thereof may bind to an epitope comprising amino acids at positions 11, 12, 13, 15 and 16 of the N-terminal extracellular domain of CCR9 isoform A. The epitope may be a discontinuous epitope comprising amino acids at positions 11, 12, 13, 15 and 16 of the N-terminal extracellular domain of CCR9 isoform A. Positions 11, 12, 13, 15 and 16 of the N-terminal extracellular domain of CCR9 isoform A may otherwise be referred to as P11, N12, M13, D15 and D16.

In some embodiments, the epitope comprises amino acids at positions 10, 11, 12, 13, 15 and 16 of the N-terminal extracellular domain of CCR9 isoform A. The epitope may be a discontinuous epitope comprising amino acids at positions 10, 11, 12, 13, 15 and 16 of the N-terminal extracellular domain of CCR9 isoform A. Position 10 may otherwise be referred to as I10.

In some embodiments, the epitope comprises amino acids at positions 10, 11, 12, 13, 15, 16 and 17 of the N-terminal extracellular domain of CCR9 isoform A. The epitope may be a discontinuous epitope comprising amino acids at positions 10, 11, 12, 13, 15, 16 and 17 of the N-terminal extracellular domain of CCR9 isoform A. Position 17 otherwise be referred to as Y17.

In some embodiments, the epitope does not comprise position 14, which may otherwise be referred to as A14, of the N-terminal extracellular domain of CCR9 isoform A.

Various methods for epitope mapping are available and known to those skilled in the art. In particular, the epitope may be as determined by a peptide scan. In certain cases, the epitope may be as determined by alanine scanning mutagenesis.

In some embodiments, the antibody of the invention is bispecific or multispecific. Preferably, the antibody of the invention is a monoclonal antibody.

In some embodiments, the anti-CCR9 antibody or antigen binding fragment thereof is humanised. The term "humanised", as used herein, will be understood to refer to derivation from a non-human antibody or antigen binding fragment thereof, typically a murine antibody, that retains the antigen-binding properties of the parent antibody or antigen binding fragment thereof, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; or (c) transplanting the entire non-human variable domains, but "cloaking" them with a human-like section by replacement of surface residues.

Methods for humanizing non-human antibodies have been described in the art. A humanised antibody may have one or more amino acid residues introduced into it from a source which is non-human. These non-human amino acid residues are often referred to as "import" residues, which are typically taken from an "import" variable domain.

Various humanisation methods are available to the skilled person. For example, the skilled person can compare the VH and VL sequences of the murine antibodies to the human germline V segment repertoire, e.g. using the tool Igblast. V segments with high homology can then be identified, and a comparison of humanness scores defined by Andrew Martin (Abhinandan, K. R. and Martin, Andrew C. R. (2007) Analyzing the 'degree of humanness' of antibody sequences, J. Mol. Biol, 369, 852-862.) included to identify segments with a high H or Z-score.

The antibody of the invention can be of any isotype. For example, the isotype may be IgM, IgD, IgG, IgE, and IgA. Preferably, the anti-CCR9 antibody or antigen binding fragment thereof comprises an IgG anti-CCR9 antibody. In some embodiments, the antibody comprises an IgG1, IgG2, IgG3 or IgG4 anti-CCR9 antibody. For example, the antibody may comprise an IgG1 anti-CCR9 antibody.

In some embodiments, the antibody or antigen binding fragment exhibits a binding affinity dissociation constant K_{D} for human CCR9 of about 1 × 10⁻⁶ M or less.

In some embodiments, the antibody or antigen binding fragment exhibits a binding affinity dissociation constant K_{D} for human CCR9 of about 1 × 10⁻⁷ M or less.

In some embodiments, the antibody or antigen binding fragment thereof exhibits a binding affinity dissociation constant K_{D} for human CCR9 of from about 1 × 10⁻⁶ M to about 1 × 10⁻¹² M. In some embodiments, the antibody or antigen binding fragment thereof exhibits a binding affinity dissociation constant K_{D} for human CCR9 of from about 1 × 10⁻⁷ M to about 1 × 10⁻¹² M. The K_{D} value may be determined by any appropriate method. In particular, the K_{D} value may be as determined by Surface Plasmon Resonance (SPR) (e.g., BIACORE). In certain cases, the binding affinity constant K_{D} may be as determined by, or inferred from, whole cell-based antibody binding determination.

The antibody or antigen binding fragment thereof may have an EC₅₀ of less than 15nM, preferably less than 5nM. In the context of the present invention, "EC₅₀" will be understood to refer to the concentration of antibody that gives half-maximal binding *in vitro.* Therefore, a lower EC₅₀ can be indicative of a greater binding affinity. EC₅₀ can be calculated by determining the concentration of bound antibody in an antibody dilution series to the target antigen. Alternatively, EC₅₀ can be calculated by determining the binding of the antibody to an immobilised target antigen in the presence of a series of soluble target antigen in a competitive ELISA format.

The present inventors have found that antibodies of the present invention advantageously exhibit decreased EC₅₀ compared to other anti-CCR9 antibodies, such as SRB1, in an ELISA format when testing binding against an immobilised human CCR9 extracellular N-terminal region peptide (SEQ ID NO: 21). SRB1 comprises a CDR-L1 comprising the amino acid sequence RSSQSLVHSNGNTYLN (SEQ ID NO: 37), a CDR-L2 comprising the amino acid sequence KVSNRFS (SEQ ID NO: 3) and a CDR-L3 comprising the amino acid sequence SQSTHFPRT (SEQ ID NO: 38). SRB1 is the humanised version of the antibody 92R, which is disclosed in WO 2015/075269.

In some embodiments, the anti-CCR9 antibody or antigen binding fragment thereof has an EC₅₀ of less than 1nM. In some embodiments, the antibody or antigen binding fragment thereof has an EC₅₀ of from about 0.5nM to about 15nM. In some embodiments, the antibody or antigen binding fragment thereof has an EC₅₀ of from about 0.5nM to about 5nM. In some embodiments, the antibody or antigen binding fragment thereof has an EC₅₀ of less than 0.6nM.

The present inventors have also advantageously found that the antibodies or antigen binding fragments thereof of the present invention retain binding capacity to human CCR9 after exposure to high temperatures (e.g. a temperature of 23°C or a temperature of 40°C). Thus, in some embodiments, the anti-CCR9 antibody or antigen binding fragment thereof binds to human CCR9 at 23°C at at least about 80% of the level of binding which is exhibited by the antibody or antigen binding fragment at 4°C. In some embodiments, the anti-CCR9 antibody or antigen binding fragment thereof binds to human CCR9 at 40°C at at least about 80% of the level of binding which is exhibited by the antibody or antigen binding fragment at 4°C. In some embodiments, the anti-CCR9 antibody or antigen binding fragment thereof binds to human CCR9 at 23°C at at least about 90%, optionally at least about 95%, of the level of binding which is exhibited by the antibody or antigen binding fragment at 4°C. In some embodiments, the anti-CCR9 antibody or antigen binding fragment thereof binds to human CCR9 at 40°C at at least about 90%, optionally at least about 95% of the level of binding which is exhibited by the antibody or antigen binding fragment at 4°C.

In some embodiments, the anti-CCR9 antibody or antigen binding fragment thereof binds to human CCR9 after incubation at 23°C for at least 24 hours at at least about 80% of the level of binding which is exhibited by the antibody or antigen binding fragment at 4°C. In some embodiments, the anti-CCR9 antibody or antigen binding fragment thereof binds to human CCR9 after incubation at 40°C for at least 24 hours at at least about 80% of the level of binding which is exhibited by the antibody or antigen binding fragment at 4°C. In some embodiments, the anti-CCR9 antibody or antigen binding fragment thereof binds to human CCR9 after incubation at 23°C for at least 24 hours at at least about 90%, optionally at least about 95%, of the level of binding which is exhibited by the antibody or antigen binding fragment at 4°C. In some embodiments, the anti-CCR9 antibody or antigen binding fragment thereof binds to human CCR9 after incubation at 40°C for at least 24 hours at at least about 90%, optionally at least about 95% of the level of binding which is exhibited by the antibody or antigen binding fragment at 4°C.

Measurement of the effect of temperature on the stability and subsequent level of binding of the antibody or antigen binding fragment thereof to human CCR9 can be achieved by *in vitro* incubation of the antibody or antigen binding fragment thereof at a temperature of about 23°C or about 40°C for at least 24 hours, optionally at least 48 or 72 hours, further optionally for about 96 hours. As a control, the antibody or antigen binding fragment thereof can be incubated *in vitro* at 4°C for the same period of time (e.g. at least 24 hours, optionally about 96 hours). After incubation, serial dilutions of the incubated antibodies can be incubated with CCR9-expressing cells (e.g. MOLT-4 cells) or CCR9 peptide for 1 hour at 4°C and bound antibodies detected, for example by flow cytometry or fluorescence imaging.

Advantageously, the present inventors have also found that antibodies of the present invention exhibit binding stability at low and high pH (pH 5 and pH 8.5, respectively), relative to a neutral pH. This is improved relative to other anti-CCR9 antibodies such as SRB1. By "neutral pH", this will be understood to refer to the pH of culture media or buffer such as PBS. A neutral pH will be considered to be a pH of from about 7 to about 7.6. Preferably, a neutral pH is about 7.4. Thus, in some embodiments, the antibody or antigen binding fragment thereof binds to human CCR9 at about pH 5 at at least about 80% of the level of binding which is exhibited by the antibody or antigen binding fragment at a neutral pH. In some embodiments, the anti-CCR9 antibody or antigen binding fragment thereof binds to human CCR9 at about pH 8.5 at at least about 80% of the level of binding which is exhibited by the antibody or antigen binding fragment at a neutral pH. In some embodiments, the anti-CCR9 antibody or antigen binding fragment thereof binds to human CCR9 at about pH 5 at at least about 90%, optionally at least about 95%, of the level of binding which is exhibited by the antibody or antigen binding fragment at a neutral pH. In some embodiments, the anti-CCR9 antibody or antigen binding fragment thereof binds to human CCR9 at about pH 8.5 at at least about 90%, optionally at least about 95% of the level of binding which is exhibited by the antibody or antigen binding fragment at a neutral pH.

The pH stability of the antibodies of the invention can be measured by various methods in the art. An exemplary method may comprise flow cytometric analysis of antibody binding after the antibody has been incubated at about pH 5 or about pH 8.5 with CCR9 expressing cells, for example MOLT-4 cells. Incubation of the antibody at about pH 5 or about pH 8.5 may occur overnight, for example for at least eight hours. Incubation at about pH 5 may be achieved by dialysis in 100 mM sodium acetate (with 150 mM NaCl), while incubation at about pH 8.5 may be achieved by dialysis in in 100 mM Tris-HCl (with 150 mM NaCl).

The present inventors have advantageously found that certain anti-CCR9 antibodies exhibit CCR9 binding that is essentially unaffected by the presence of the ligand CCL25. This contrasts with an anti-CCR9 antibody such as "3C3" disclosed in WO00/53635 which displays significant sensitivity to the presence of CCL25. Indeed, at physiologically relevant concentrations of CCL25 the binding of the antibody "3C3" to CCR9 is effectively abolished. In some embodiments the antibody or antigen-binding fragment thereof exhibits CCR9 specific binding in the presence of 400nm concentration of human CCL25, as measured by flow cytometry.

The present inventors have also observed that certain anti-CCR9 antibodies are internalised intracellularly upon binding to a CCR9-expressing cell. Thus, the antibody or antigen-binding fragment thereof may be internalized by CCR9-expressing cells. As the skilled person will appreciate, various methods of measuring antibody internalization are known and available to those skilled in the art. For example, the present inventors used pHrodo labelling of the antibodies to monitor internalization. pHrodo dyes are non-fluorescent at a neutral pH and become fluorescent in the intracellular acidic environment. Fluorescence can then be measured, for example, by flow cytometry.

In some embodiments, the melting point of the antibody or antigen-binding fragment thereof is of from about 60°C to about 70°C. The melting point of the antibody or antigen-binding fragment thereof may be of from about 60° to about 67°C. In some embodiments the melting point of the antibody or antigen-binding fragment thereof may be of from about 61°C to about 67°C. The melting point may otherwise be referred to herein as the aggregation point and can be measured using dynamic light scattering (DLS).

The present inventors have observed that certain anti-CCR9 antibodies exhibit binding stability after *in vitro* incubation in 50% human plasma for at least about 24 hours. Therefore, in some embodiments, the antibody or antigen-binding fragment thereof binds to human CCR9 after incubation in 50% human plasma at at least about 50% of the level of binding exhibited by the antibody or antigen binding fragment without incubation in 50% human plasma. Incubation in 50% human plasma may be for at least about 24 hours, at least about 72 hours or at least about 168 hours. In some embodiments, the antibody or antigen-binding fragment thereof binds to human CC49 after incubation in 50% human plasma at at least about 80% of the level of binding exhibited by the antibody or antigen binding fragment without incubation in 50% human plasma.

In some embodiments the antibody or antigen-binding fragment thereof exhibits CCR9+ lymphocyte depletion. The present inventors have, for example, observed antibody-dependent cellular cytotoxicity (ADCC) utilizing antibodies of the invention.

### Antibody drug conjugates (ADCs) and/or fusion proteins

In some embodiments, the antibody or antigen-binding fragment thereof of the present invention is conjugated or fused to a further compound, such as a therapeutic agent, a toxin or the like. Such an antibody-compound conjugate may otherwise be referred to as an immunoconjugate or fusion protein.

Preferably, the antibody or antigen-binding fragment thereof of the present invention is comprised in an antibody-drug conjugate (ADC). ADCs are therapeutic agents which comprise an antibody, in this instance an antibody or antigen-binding fragment of the invention, conjugated or fused to a therapeutic agent, which may otherwise be referred to herein as a drug. The drug is typically a cytotoxic or chemotherapy agent. The ADC may comprise the formula A-L-C, wherein A comprises the antibody or antigen-binding fragment thereof, L comprises a ligand and C comprises the drug, preferably a cytotoxic or chemotherapy agent.

Suitable therapeutic agents for forming immunoconjugates of the present invention include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, mitoxantrone, mithramycin, actinomycin D, 1-ehydrotestosterone, glucocorticoids, procaine, tetracaine, lidocaine, propranolol, and puromycin; calicheamicin or analogs or derivatives thereof; antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a .k.a. rachelmycin), or analogs or derivatives of CC- 1065), antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (e.g., tubulin-inhibitors) such as monomethyl auristatin E, monomethyl auristatin F, or other analogs or derivatives of dolastatin 10; diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin. See, for example, Pastan et al., Cell 47, 641 (1986) and Goldenberg, Calif. A Cancer Journal for Clinicians 44, 43 (1994).

In some embodiments, the antibody or antigen-binding fragment thereof is conjugated to a therapeutic agent selected from the group consisting of a cytotoxin, a radionuclide, an immunosuppressant, a chemotherapeutic drug and a cytokine.

### Further anti-CCR9 antibodies or antigen binding fragments thereof

Also provided by the present invention is an anti-CCR9 antibody or antigen binding fragment thereof comprising a variable light chain comprising SEQ ID NO:39, wherein SEQ ID NO: 39 comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In some embodiments, the CDR-L1 comprises the amino acid sequence RSSQSLVHSNGITFLH (SEQ ID NO: 5) and the CDR-L3 comprises the amino acid sequence SQTTHVPWT (SEQ ID NO: 7)

The antibody or antigen binding fragment thereof may be as defined above in relation to the first aspect. In particular, the CDR-Hs and the variable heavy chain may be as defined above in relation to the first aspect.

The present invention also provides an anti-CCR9 antibody or antigen binding fragment thereof comprising a variable light chain comprising SEQ ID NO: 40, wherein SEQ ID NO: 40 comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

In some embodiments, the CDR-L1 comprises the amino acid sequence RSSQSLLHSNGNTFLH (SEQ ID NO: 6) and the CDR-L3 comprises the amino acid sequence SQTTHLPWT (SEQ ID NO: 8).

The antibody or antigen binding fragment thereof may be as defined above in relation to the first aspect. In particular, the CDR-Hs and the variable heavy chain may be as defined above in relation to the first aspect.

According to a further aspect of the invention, there is provided an anti-CCR9 antibody or antigen binding fragment thereof, wherein the anti-CCR9 antibody or antigen binding fragment thereof binds to a discontinuous epitope comprising amino acids at positions 11, 12, 13, 15 and 16 of the of the N-terminal extracellular domain of CCR9 isoform A. Positions 11, 12, 13, 15 and 16 of the N-terminal extracellular domain of CCR9 isoform A may otherwise be referred to as P11, N12, M13, D15 and D16. The anti-CCR9 antibody or antigen fragment thereof may be as defined above in relation to the first aspect of the invention.

For example, in some embodiments, the epitope comprises amino acids at positions 10, 11, 12, 13, 15 and 16 of the N-terminal extracellular domain of CCR9 isoform A.

In some embodiments, the epitope comprises amino acids at positions 10, 11, 12, 13, 15, 16 and 17 of the N-terminal extracellular domain of CCR9 isoform A.

In some embodiments, the epitope does not comprise position 14, which may otherwise be referred to as A14, of the N-terminal extracellular domain of CCR9 isoform A.

### Polynucleotides

According to a further aspect, there is provided a polynucleotide comprising a nucleotide sequence encoding the anti-CCR9 antibody or antigen binding fragment thereof as defined in accordance with any of the above aspects of the invention.

A polynucleotide, such as a nucleic acid, is a polymer comprising a plurality of nucleotides. The nucleotides can be naturally occurring or artificial.

A nucleotide typically contains a nucleobase, a sugar and at least one linking group, such as a phosphate, 2'O-methyl, 2' methoxy-ethyl, phosphoramidate, methylphosphonate or phosphorothioate group. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar and the nucleobase together form a nucleoside. Preferred nucleosides include, but are not limited to, adenosine, guanosine, 5-methyluridine, uridine, cytidine, deoxyadenosine, deoxyguanosine, thymidine, deoxyuridine and deoxycytidine. The nucleosides may be adenosine, guanosine, uridine and cytidine.

The nucleotides are typically ribonucleotides or deoxyribonucleotides. The nucleotides may be deoxyribonucleotides. The nucleotides typically contain a monophosphate, diphosphate or triphosphate. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), 5-methylcytidine monophosphate, 5-methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2'-deoxycytidine monophosphate, 5-methyl-2'-deoxycytidine diphosphate, 5-methyl-2'-deoxycytidine triphosphate, 5-hydroxymethyl-2'-deoxycytidine monophosphate, 5-hydroxymethyl-2'-deoxycytidine diphosphate and 5-hydroxymethyl-2'-deoxycytidine triphosphate. The nucleotides may be selected from AMP, UMP, GMP, CMP, dAMP, dTMP, dGMP or dCMP. In some embodiments, the nucleotides are selected from dAMP, dTMP, dGMP or dCMP.

The nucleotides may contain additional modifications. In particular, suitable modified nucleotides include, but are not limited to, 2'amino pyrimidines (such as 2'-amino cytidine and 2'-amino uridine), 2'-hyrdroxyl purines (such as , 2'-fluoro pyrimidines (such as 2'-fluorocytidine and 2'fluoro uridine), hydroxyl pyrimidines (such as 5'-α-P-borano uridine), 2'-O-methyl nucleotides (such as 2'-O-methyl adenosine, 2'-O-methyl guanosine, 2'-O-methyl cytidine and 2'-O-methyl uridine), 4'-thio pyrimidines (such as 4'-thio uridine and 4'-thio cytidine) and nucleotides have modifications of the nucleobase (such as 5-pentynyl-2'-deoxy uridine, 5-(3-aminopropyl)-uridine and 1,6-diaminohexyl-N-5-carbamoylmethyl uridine).

One or more nucleotides in the polynucleotide may be modified, for instance with a label or a tag. The label may be any suitable label which allows the nucleotides to be detected. Suitable labels include, but are not limited to, fluorescent molecules, radioisotopes, e.g. 1251, 35S, enzymes, antibodies, antigens, other polynucleotides and ligands such as biotin.

The polynucleotide may comprise deoxyribonucleic acid (DNA) or ribonucleic acid (RNA).

Unless otherwise indicated, a particular polynucleotide sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with a different base, mixed-base and/or deoxyinosine residues (Batzer et al., 1991, Nucleic Acid Res. 19:5081; Ohtsuka et al., 1985, J. Biol. Chem. 260:2605-2608; and Rossolini et al., 1994, Mol. Cell. Probes 8:91-98).

Substitutions may be used for the practices of codon optimisation and codon wobble, both of which are known to those skilled in the art. Thus, it will be appreciated that codon-optimised and codon-wobbled polynucleotides are also envisaged. In some embodiments, the polynucleotide is codon-optimised for human expression.

The polynucleotide can be produced by de novo solid-phase DNA synthesis or by PCR mutagenesis of an existing sequence. Direct chemical synthesis of polynucleotides can be accomplished by methods known in the art, such as the phosphotriester method of Narang et al., 1979, Meth. Enzymol. 68:90; the phosphodiester method of Brown et al., 1979, Meth. Enzymol. 68:109; the diethylphosphoramidite method of Beaucage et al., 1981, Tetra. Lett., 22:1859; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., 1991, Nucleic Acids Res. 19:967; and Eckert et al., 1991, PCR Methods and Applications 1:17.

### Vectors

The invention also provides a vector comprising the polynucleotide of the above aspect.

The vector may be an expression vector. The vector may be viral or non-viral.

Non-viral vectors and systems include plasmids, episomal vectors, typically with an expression cassette for expressing a protein or RNA, and human artificial chromosomes (see, e.g., Harrington et al., 1997, Nat Genet. 15:345). For example, non-viral vectors useful for expression of the antibody or antigen-binding fragment thereof of the invention in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, 1995, Annu. Rev. Microbiol. 49:807; and Rosenfeld et al., 1992, Cell 68: 143.

In some embodiments, the vector is a retroviral or lentiviral vector. Optionally, the vector is an SFG retroviral vector. In some embodiments the vector is a lentiviral vector. Lentiviral vectors include self-inactivating lentiviral vectors (so-called SIN vectors).

The choice of vector depends on the intended host cells in which the vector is to be expressed. Expression vectors for mammalian host cells may include expression control sequences, such as an origin of replication, a promoter, and an enhancer (see, e.g., Queen, et al., 1986, Immunol. Rev. 89:49-68), and processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pollll promoter, the constitutive MPS V promoter, the tetracycline-inducible CMV promoter (such as the human immediate-early CMV promoter), the constitutive CMV promoter, the EF1 alpha promoter, the phosphoglycerate kinase (PGK) promoter and promoter-enhancer combinations known in the art.

Cultures of transformed organisms can be expanded under non-inducing conditions without biasing the population for coding sequences whose expression products are better tolerated by the host cells. In addition to promoters, other regulatory elements may also be required or desired for efficient expression of the antibody or antigen binding fragment thereof of the invention. These elements may include an ATG initiation codon and adjacent ribosome binding site or other sequences. In addition, the efficiency of expression may be enhanced by the inclusion of enhancers appropriate to the cell system in use (see, e.g., Scharf et al., 1994, Results Probl. Cell Differ. 20:125; and Bittner et al., 1987, Meth. Enzymol., 153:516). For example, the SV40 enhancer or CMV enhancer may be used to increase expression in mammalian host cells.

### Cells

The present invention also provides a cell comprising the anti-CCR9 antibody or antigen binding fragment thereof, the polynucleotide or the vector as defined in any of the aspects above.

The cell may be prokaryotic or eukaryotic.

Cells suitable include, but are not necessarily limited to, mammalian, plant, insect, fungal and bacterial cells. Bacterial cells include, without limitation, cells from Gram positive bacteria such as species of the genus Bacillus, Streptomyces and Staphylococcus and Gram-negative bacterial cells such as cells of the genus Escherichia and Pseudomonas. Fungal cells preferably include yeast cells such as Saccharomyces, Pichia pastoris and Hansenula polymorpha. Insect cells include, but are not necessarily limited to Drosophila cells and Sf9 cells. Plant cells include, among others, cells of crop plants such as cereals, medicinal, ornamental or bulbs. Mammalian cells suitable for the present invention include epithelial cell lines, osteosarcoma cell lines, neuroblastoma cell lines, epithelial carcinomas, glial cells, hepatic cell lines, CHO (Chinese Hamster Ovary) cells, COS cells, BHK cells, HeLa cells, 911 cells, AT1080 cells, A549 cells, 293 and 293T cells, PER.C6 cells, NTERA-2 human ECCs cells, D3 cells of the mESCs line, human embryonic stem cells such as HS293, hMSCs and BGV01, SHEF1, SHEF2 and HS181, NIH3T3 cells, REH and MCF-7 cells.

The cell may comprise a hybridoma cell. The term "hybridoma", as used herein, refers to a hybrid cell line formed by fusing a specific antibody-producing B cell with a myeloma (B cell cancer) cell that is selected for its ability to grow in tissue culture and for an absence of antibody chain synthesis. The antibodies produced by the hybridoma are usually of a single specificity and are therefore monoclonal antibodies (in contrast to polyclonal antibodies). The production of monoclonal antibodies was invented by César Milstein and Georges J. F. Köhler in 1975 (Köhler and Milstein, 1975, Nature 256:495- 7).

In some embodiments, the cell is an immune cell, such as a T or B cell. In some embodiments, the cell is a T cell. In some embodiments, the cell is a CAR cell. In some embodiments, the cell is a CAR T-cell. Preferably, the CAR comprises the CDR regions of the anti CCR9 antibody or antigen binding fragment thereof of the invention. For example, the CAR may comprise a variable light chain comprising SEQ ID NO:1 or a variant thereof having at least 95% sequence identity to SEQ ID NO:1, wherein the variable light chain comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid. In some embodiments the CAR further comprises a CDR-H1 comprising the amino acid sequence GFTFSXYWMX (SEQ ID NO: 9), a CDR-H2 comprising the amino acid sequence EIRSKSXNYATHYXESXXG (SEQ ID NO: 10) and a CDR-H3 comprising the amino acid sequence XYSPY (SEQ ID NO: 11), wherein X can be any amino acid.

### Pharmaceutical compositions

According to a further aspect, the present invention provides a pharmaceutical composition comprising the anti-CCR9 antibody or antigen binding fragment thereof, the ADC, the polynucleotide, the cell or the vector as defined in any of the above aspects and a pharmaceutically or physiologically acceptable diluent and/or carrier.

The carrier and/or diluent is generally selected to be suitable for the intended mode of administration and can include agents for modifying, maintaining, or preserving, for example, the pH, osmolarity, viscosity, clarity, colour, isotonicity, odour, sterility, stability, rate of dissolution or release, adsorption, or penetration of the composition. Typically, these carriers and/or diluents include aqueous or alcoholic/aqueous solutions, emulsions, or suspensions, including saline and/or buffered media.

Suitable further agents for inclusion in the pharmaceutical compositions may include, but are not limited to, amino acids (such as glycine, glutamine, asparagine, arginine, or lysine), antimicrobials, antioxidants (such as ascorbic acid, sodium sulphite, or sodium hydrogen-sulphite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as free serum albumin, gelatin, or immunoglobulins), colouring, flavouring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as Polysorbate 20 or Polysorbate 80; Triton; tromethamine; lecithin; cholesterol or tyloxapal), stability enhancing agents (such as sucrose or sorbitol), tonicity enhancing agents (such as alkali metal halides, such as sodium or potassium chloride, or mannitol sorbitol), delivery vehicles, excipients and/or pharmaceutical adjuvants.

The carrier and/or diluent may be a parenteral, optionally intravenous vehicle. Suitable parenteral vehicles may include sodium chloride solution, Ringer's dextrose, dextrose and sodium chloride and lactated Ringer's. Suitable physiologically-acceptable thickeners such as carboxymethylcellulose, polyvinylpyrrolidone, gelatin and alginates may be included. Intravenous vehicles include fluid and nutrient replenishers and electrolyte replenishers, such as those based on Ringer's dextrose. In some cases, one might include agents to adjust tonicity of the composition, for example, sugars, polyalcohols such as mannitol, sorbitol, or sodium chloride in a pharmaceutical composition. For example, in many cases it is desirable that the composition is substantially isotonic. Preservatives and other additives, such as antimicrobials, antioxidants, chelating agents, and inert gases, may also be present. The precise formulation will depend on the route of administration. Additional relevant principles, methods and components for pharmaceutical formulations are well known (see, e.g., Allen, Loyd V. Ed, (2012) Remington's Pharmaceutical Sciences, 22nd Edition).

A pharmaceutical composition of the present invention can be administered by one or more routes of administration using one or more of a variety of methods known in the art. As will be appreciated by the skilled person, the route and/or mode of administration will vary depending upon the desired results. Routes of administration for pharmaceutical compositions of the invention may include intravenous, intramuscular, intradermal, intraperitoneal, intrapleural, subcutaneous, intratumoural, spinal, or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, intratumoural, intrapleural and intra-sternal injection and infusion. In some embodiments, the pharmaceutical composition is administered intratumourally. In other embodiments, administration is intrapleural or intraperitoneal. When parenteral administration is contemplated, the pharmaceutical compositions are usually in the form of a sterile, pyrogen-free, parenterally acceptable composition. A particularly suitable vehicle for parenteral injection is a sterile, isotonic solution, properly preserved. The pharmaceutical composition can be in the form of a lyophilizate, such as a lyophilized cake.

Alternatively, the pharmaceutical composition of the invention can be administered by a nonparenteral route, such as a topical, epidermal, or mucosal route of administration, for example, intranasally, orally, or sublingually.

In some embodiments, the pharmaceutical composition is for subcutaneous administration. Typically, the pharmaceutical compositions for subcutaneous administration contain suitable stabilizers (e.g., amino acids, such as methionine, and or saccharides such as sucrose), buffering agents and tonicifying agents. Alternatively, the pharmaceutical composition may be for intravenous administration.

### Kits

According to a further aspect, there is provided a kit comprising the anti-CCR9 antibody or antigen binding fragment thereof, the ADC, the polynucleotide, the vector, the cell or the pharmaceutical composition as defined according to any of the above aspects.

The kit may further comprise instructions for use. In some embodiments, the antibody or antigen binding fragment thereof is provided in an aqueous solution, optionally buffered solution. In some embodiments, the antibody or antigen binding fragment thereof is provided at a temperature of 4°C or less. For example the antibody or antigen binding fragment may be provided at a temperature of -20°C or less.

### Methods of producing the antibody or antigen binding fragment thereof

Also provided by the present invention is a method for producing the antibody or antigen binding fragment of the invention. The method comprises culturing a cell as defined in the above aspect under conditions permitting expression of said antibody or antigen binding fragment thereof.

The method may comprise:
a) Introducing a polynucleotide or vector as defined in the above aspects into a cell as defined in the above aspect; and
b) Culturing the cell under conditions permitting expression of said antibody or antigen binding fragment thereof.

The cell may be as defined above. For example, the cell may be prokaryotic or eukaryotic. The cell may be a mammalian cell. For example, the cell may be a HEK-293 cell or a CHO cell. The cell may be an immune cell, for example a T-cell, such as a CAR T-cell.

Introduction of a polynucleotide or vector into the cell may otherwise be referred to as genetic engineering of the cell. The genetic engineering of cells can be carried out according to standard cloning and expression techniques, which are known in the art (e.g., as described in Sambrook, J., Fritsh, E. F., and Maniatis, T. Molecular Cloning: A Laboratory Manual 2nd, ed., Cold Spring Harbor Laboratory, Cold Spring Harbor Laboratory Press, Cold Spring Harbor, N.Y., 1989). The vector of the invention may be introduced into the cell using such techniques.

For example, introduction may comprise transducing or transfecting the polynucleotide or vector into the cell.

The conditions for optimising the culture of said cell will depend on the cell used. If desired, the method for producing the antibody or antigen binding fragment thereof of the invention further includes the isolation and purification of said antibody or antigen binding fragment. For example, purification may comprise using protein A affinity chromatography.

In some embodiments the method is a method for producing the antibody drug conjugate of the invention. The method may further comprise conjugating, fusing or linking a drug as described herein to the antibody or antigen binding fragment thereof. Various suitable conjugates and linkers, as well as methods of conjugating, fusing and linking, are known to the skilled person and are commercially available.

### Methods of diagnosis

According to a further aspect, the present invention provides a method of diagnosing a disease associated with elevated CCR9 expression in a subject, the method comprising:
a) contacting the antibody or antigen binding fragment thereof of the invention with a sample comprising cells from the subject;
b) detecting and/or quantifying CCR9 expression in the subject sample;
c) comparing the level of CCR9 expression detected in the subject sample with the level of CCR9 expression detected in a control sample;
d) correlating the comparison obtained in step c) with the presence of a disease associated with elevated CCR9 expression.

In some embodiments, the subject is diagnosed with the disease when the comparison in step c) indicates an increased level of CCR9 expression in the subject sample compared to the level of CCR9 expression in the control sample. In some embodiments, the increased level of CCR9 expression may be at least 1.5x the level of CCR9 expression in the control sample. In some embodiments, the increased level of CCR9 expression may be at least 2x, optionally at least 3x, further optionally at least 5x the level of CCR9 expression in the control sample. In some embodiments, the increased level of CCR9 expression may comprise an increase of at least 25%, at least 30%, at least 40%, at least 50%, at least 60%, at least 70%, at least 80%, at least 90%, at least 100%, at least 110%, at least 120%, at least 150%, at least 200% or more in respect to the control sample.

The antibody or antigen binding fragment thereof may be as defined herein.

The disease may be selected from the group consisting of cancer, Crohn's disease, inflammatory bowel disease, colitis, ulcerative colitis, liver fibrosis and acute liver inflammation. In some embodiments, the disease is cancer. In some embodiments, the disease is inflammatory bowel disease.

The cancer may be selected from ovarian cancer, prostate cancer, breast cancer, melanoma, T-cell acute lymphoblastic leukaemia (T-ALL), pancreatic cancer, colorectal cancer, lung cancer, circulating cells from a solid tumour (CTCs), T-cell lineage lymphomas and acute myeloid leukaemia (AML).

The breast cancer may comprise metastatic breast cancer. The lung cancer may comprise non small-cell lung carcinoma (NSCLC), for example squamous lung cancer or lung adenocarcinoma.

The subject may be a mammal. Optionally, the subject is a human, horse, dog or cat. In some embodiments, the subject is human.

The term "sample", as used herein, includes different types of biological fluids, sections of tissues of the affected organs, etc. Illustrative, non-limiting examples of said samples include different types of biological fluids, such as peritoneal fluid, pleural fluid, synovial fluid, urine, saliva, blood, semen, serum, etc. These biological fluid samples can be obtained by any conventional method known by persons having ordinary skill in the art. Alternatively, the sample can also be a section of an affected organ tissue sample, for example from the lymphoid gland, breast, prostate, skin, small bowel, large, bowel, pancreas, ovary, lung, bladder, kidney, etc., which can be obtained by any conventional method, for example by means of biopsy, cystoscopy, surgical resection, etc., as well as frozen sections taken for histologic purposes.

In some embodiments, the sample is a tumour sample.

The antibody or antigen binding fragment thereof can be labelled or not labelled. The term "detectable label" or "labelling agent", as used herein, refers to a molecular label which allows the detection, localization and/or identification of the molecule to which it is attached, using suitable procedures and equipment for detection, for example by spectroscopic, photochemical, biochemical, immunochemical or chemical means.

Labelling agents that are suitable for labelling the antibody or antigen binding fragment thereof include radionuclides, enzymes, fluorophores, chemiluminescent reagents, enzyme substrates or cofactors, enzyme inhibitors, particles, dyes and derivatives, and the like. As the person skilled in the art will understand, antibodies that are not labelled need to be detected with an additional reagent, for example, a secondary antibody that is labelled, which will be labelled. This is particularly useful in order to increase the sensibility of the detection method, since it allows the signal to be amplified.

There is a wide range of conventional assays that can be used in the present invention which use an antibody of the invention that is not labelled (primary antibody) and an antibody of the invention that is labelled (secondary antibody); these techniques include Western blot or immunoblot, ELISA (Enzyme-Linked Immunosorbent Assay), RIA (Radioimmunoassay), competitive EIA (Competitive Enzyme Immunoassay), DAS- ELISA (Double Antibody Sandwich-ELISA), immunocytochemical and immunohistochemical techniques, flow cytometry or multiplex detection techniques based on using protein microspheres, biochips or microarrays which include the antibody of the invention. Other ways of detecting and quantifying CCR9 using the antibody of the invention may include affinity chromatography techniques, ligand binding assays or lectin binding assays.

The term "control sample" or "reference sample", as used herein, refers to a sample that comprises undetectable levels of CCR9 expression, or comprises a level of CCR9 expression considered to be a normal physiological level. In some embodiments, the control sample is from a healthy subject, although it may also be obtained from other subjects, including subjects who do not have a clinical history of a disease or condition associated with elevated CCR9 expression, the same subject but from a tissue or part thereof which is under a normal physiological condition or healthy. In some embodiments, the control sample is a sample from an individual who does not have a clinical history of a disease or condition associated with elevated CCR9 expression.

In embodiments where the subject is diagnosed with the disease, the method may further comprise step e) comprising a method of treating the disease in the subject. The method of treating the disease may be as defined according to the aspect below.

### Methods of treatment or prevention

Also provided is a method of treating or preventing a disease in a subject, wherein the method comprises administering to the subject the antibody or antigen binding fragment thereof, the ADC, the polynucleotide, the vector, the cell or the pharmaceutical composition of the invention.

The disease may be selected from the group consisting of cancer, Crohn's disease, inflammatory bowel disease, colitis, ulcerative colitis, liver fibrosis and acute liver inflammation. In some embodiments, the disease is cancer. In some embodiments, the disease is inflammatory bowel disease.

The cancer may be selected from ovarian cancer, prostate cancer, breast cancer, melanoma, T-cell acute lymphoblastic leukaemia (T-ALL), pancreatic cancer, colorectal cancer, lung cancer, circulating cells from a solid tumour (CTCs), T-cell lineage lymphomas and acute myeloid leukaemia (AML).

The breast cancer may comprise metastatic breast cancer. The lung cancer may comprise non small-cell lung carcinoma (NSCLC), for example squamous lung cancer or lung adenocarcinoma.

The method typically comprises administering a therapeutically effective amount or a prophylactically effective amount of the antibody or antigen binding fragment thereof, the ADC, the polynucleotide, the vector, the cell or the pharmaceutical composition of the invention. A therapeutically effective amount is an amount which ameliorates one or more symptoms, such as all the symptoms, of the disease and/or abolishes one or more symptoms, such as all the symptoms, of the disease. The therapeutically effective amount preferably cures the disease. A prophylactically effective amount is an amount which prevents the onset of the disease and/or prevents the onset of one or more symptoms, such as all the symptoms, of the disease. The prophylactically effective amount preferably prevents the subject from developing the disease. Suitable amounts are discussed in more detail below.

The antibody or antigen binding fragment thereof, the ADC, the polynucleotide, the vector, the cell or the pharmaceutical composition of the invention may be administered to a subject that displays symptoms of disease. The antibody or antigen binding fragment thereof, the ADC, the polynucleotide, the vector, the cell or the pharmaceutical composition of the invention may be administered to a subject that is asymptomatic, i.e. does not display symptoms of disease. The antibody or antigen binding fragment thereof, the ADC, the polynucleotide, the vector, the cell or the pharmaceutical composition of the invention may be administered when the subject's disease status is unknown or the subject is expected not to have a disease. The antibody or antigen binding fragment thereof, the ADC, the polynucleotide, the vector, the cell or the pharmaceutical composition of the invention may be administered to a subject that is predisposed, such as genetically predisposed, to developing the disease.

The subject may be a mammal. Optionally, the subject is a human, horse, dog or cat. In some embodiments, the subject is human.

In some embodiments the subject is a mammal (preferably a human) who has been diagnosed as having, or as being at risk of developing, a cancer. In some embodiments, the subject has been or is being treated with anti-cancer therapy (e.g. with an anti-cancer drug treatment, surgical treatment and/or radiotherapy). In some embodiments, the cancer of the subject may have relapsed, spread and/or developed resistance to a first-line therapy.

In some embodiments the cancer of the subject is advanced, metastatic and/or refractory cancer.

The subject may have been pre-treated with a chemotherapeutic agent.

In embodiments where the subject is a mammal who has been diagnosed as having, or as being at risk of developing a cancer, the administration of the antibody or antigen binding fragment thereof, the ADC, the polynucleotide, the vector, the cell or the pharmaceutical composition of the invention to the subject may result in a decrease in tumour size of about 10%, about 20%, about 30%, about 40%, about 50%, about 60%, about 70%, about 80%, about 90%, or even about 100%, when compared to an untreated tumour.

Any suitable form of administration may be used for the antibody or antigen binding fragment thereof, the ADC, the polynucleotide, the vector, the cell or the pharmaceutical composition of the invention. For example, the antibody or antigen binding fragment thereof, the ADC, the polynucleotide, the vector, the cell or the pharmaceutical composition of the invention may be administered subcutaneously, intranasally, orally, topically, intraperitoneally or intravenously. In some embodiments, the antibody or antigen binding fragment thereof, the ADC, the polynucleotide, the vector, the cell, or the pharmaceutical composition of the invention may be intraperitoneally administered.

In a further aspect, the present invention provides the antibody or antigen binding fragment thereof, the ADC, the polynucleotide, the vector, the cell or the pharmaceutical composition of the invention for use in any of the therapeutic methods described above. Thus, also provided is the antibody or antigen binding fragment thereof, the ADC, the polynucleotide, the vector, the cell or the pharmaceutical composition of the invention for use in the treatment or prevention of a disease. The disease may be selected from cancer, Crohn's disease, inflammatory bowel disease, colitis, ulcerative colitis, liver fibrosis and acute liver inflammation. In particular, the invention provides the antibody or antigen binding fragment thereof, the ADC, the polynucleotide, the vector, the cell or the pharmaceutical composition of the invention for use in the treatment or prevention of cancer. The cancer may be as defined for the method of treatment aspect.

Also provided is the use of the antibody or antigen binding fragment thereof, the ADC, the polynucleotide, the vector, the cell or the pharmaceutical composition of the invention for the manufacture of a medicament for the treatment or prevention of a disease. The disease may be as defined above in relation to the method of treatment or medical use aspects. Optionally, the disease is cancer. Further provided is use of the antibody or antigen binding fragment thereof, the ADC, the polynucleotide, the vector, the cell or the pharmaceutical composition of the invention for the treatment or prevention of a disease. The disease may be as defined above in relation to the method of treatment of medical use aspects. For example, the disease may comprise cancer.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

All documents mentioned in this text are incorporated herein by reference.

### Examples

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press.

### Example 1

### Production and quality control of humanized anti-CCR9 antibodies

Two pools of murine antibody candidates targeting CCR9 were generated using CCR9 N-terminal peptide immunization by intraperitoneal injection.

The spleen was excised and spleen cells were fused with P3U1 cell line. Culture supernatants were screened by ELISA and then flow cytometry to select hybridomas that were producing anti-CCR9 mAbs.

Murine anti-CCR9 antibodies were screened for affinity to CCR9. The top binding antibodies (IAb01, IAb02, C9Mab-16 and C9Mab-19) were selected for humanization and subsequent characterization.

Humanized variants of the four selected antibodies were generated by CDR grafting, including one variant for IAb01 (huIAb01) and two variants for each of the other antibodies (huIAb02, huC9Mab-16, huC9Mab-19). One humanized VH domain was combined with two different humanized VL domains (VL1, VL2) based on two different human VL germline genes (Fig. 1).

For clones 16 and 19 one VH segment (IGHV3-73*01) was used but 2 different VL segments (either IGKV2-30*01 or IGKV2-29*01 and IGKV1-30*01) giving the humanized sequences VL1 and VL2, respectively. Of note, GKV2-30*01 and GKV2-29*01 have a very negative z-score for humanness, while IGKV1-30*01) has a very high positive z-score, i.e. is defined as very human and thus favorable. Of note IGKV1-30*01 is less homologous to the parental antibodies 16 and 19 as IGKV2-30*01 or IGKV2-29*01. All sequences were further analysed for canonical structure before and after CDR grafting and 2 mutations were introduced into the N-terminal region (FR1) of IGKV1-30*01 derived sequences to obtain the same canonicals as in the parental sequences.

huC9Mab-16 VL2 comprises a variable light chain comprising SEQ ID NO:1. SEQ ID NO: 1 comprises a CDR-L1 comprising the amino acid sequence RSSQSLVHSNGITFLH (SEQ ID NO: 5), a CDR-L2 comprising the amino acid sequence KVSNRFS (SEQ ID NO: 3) and a CDR-L3 comprising the amino acid sequence SQTTHVPWT (SEQ ID NO: 7). huC9Mab-16 VL2 also comprises a variable heavy chain (SEQ ID NO: 18) comprising a CDR-H1 comprising the amino acid sequence GFTFSDYWMN (SEQ ID NO: 12), a CDR-H2 comprising the amino acid sequence EIRSKSKNYATHYEESVRG (SEQ ID NO: 14) and a CDR-H3 comprising the amino acid sequence FYSPY (SEQ ID NO: 16). huC9Mab-19 VL2 comprises a variable light chain comprising SEQ ID NO: 20. SEQ ID NO: 20 comprises a CDR-L1 comprises amino acid sequence RSSQSLLHSNGNTFLH (SEQ ID NO: 6), a CDR-L2 comprising the amino acid sequence KVSNRFS (SEQ ID NO: 3), and a CDR-L3 comprising the amino acid sequence SQTTHLPWT (SEQ ID NO: 8). huC9Mab-19 VL2 also comprises a variable heavy chain (SEQ ID NO: 19) comprising a CDR-H1 comprising the amino acid sequence GFTFSNYWMD (SEQ ID NO: 13), a CDR-H2 comprising the amino acid sequence EIRSKSNNYATHYAESMKG (SEQ ID NO: 15) and a CDR-H3 comprising the amino acid sequence YYSPY (SEQ ID NO: 17).

A direct comparison of the sequences of the variable domains of the anti-CCR9 antibodies demonstrated differences in the CDR regions between the different antibodies. The anti-CCR9 antibody SRB1 or SRB10 (92R, as disclosed in WO 2015/075269) has been included in all the different experiments as a positive control (Fig. 1). SRB1 comprises a CDR-L1 comprising the amino acid sequence RSSQSLVHSNGNTYLN (SEQ ID NO: 37), a CDR-L2 comprising the amino acid sequence KVSNRFS (SEQ ID NO: 3) and a CDR-L3 comprising the amino acid sequence SQSTHFPRT (SEQ ID NO: 38).

The humanized anti-CCR9 antibodies were successfully produced using HEK293-6E cells for transfection and using Protein A affinity chromatography for purification. The humanized anti-CCR9 antibodies were analyzed by SDS-PAGE using reducing or non-reducing conditions (Fig. 2). SDS-PAGE under reducing conditions revealed two bands at approximately 55 kDa and in the range of 26 kDa to 24 kDa for the anti-CCR9 antibodies representing the heavy and light chain of the antibodies, respectively. Under non-reducing conditions, one major peak was detected above 170 kDa for all humanized anti-CCR9 antibodies representing the intact molecule containing two heavy chains and two light chains. Thus, SDS-PAGE demonstrated purity and correct composition of all the IgG antibodies.

In addition, all humanized anti-CCR9 antibodies were also subjected to analytical size-exclusion chromatography using HPLC (Fig. 3). Integrity of all humanized anti-CCR9 antibodies was confirmed by a major peak using SEC-HPLC analysis.

### Example 2

### Binding analysis of the humanized antibodies

Binding of the humanized anti-CCR9 antibodies was analyzed by ELISA using a biotinylated peptide corresponding to the human CCR9 extracellular N-terminal region (TPTDFTSPIPNMADDYGSESTSS, SEQ ID NO: 22). SEQ ID NO: 22 comprises amino acids 2-24 of the human CCR9 extracellular N-terminal region, and so comprises SEQ ID NO: 21 (amino acids 2-22 of the human CCR9 extracellular N-terminal region).

ELISA plates were coated with the biotinylated peptide overnight at 4°C. ELISA plates were blocked and washed, and serial dilution of the antibodies were added to the wells and incubated for 1 hour at room temperature. Antibody detection was performed with anti-human Fc antibody, measuring absorption at 450 nm (Fig. 4). EC₅₀ values of different anti-CCR9 antibodies to the N-terminal peptide are listed in Table 2.

**Table 2: EC₅₀ values of humanized anti-CCR9 antibodies determined by ELISA binding to CCR9 N-terminal peptide 1 (TPTDFTSPIPNMADDYGSESTSS, SEQ ID NO: 22). Mean ± SD, n=2.**

| **Antibody** | **EC₅₀ [nM]** |
|---|---|
| **SRB1** | 19.17 |
| **huC9Mab16-VL1** | 759.9 |
| **huC9Mab16-VL2** | 0.546 |
| **huC9Mab19-VL1** | 8.605 |
| **huC9Mab19-VL2** | 14.07 |

The top binding antibodies (huC9Mab-16 and huC9Mab-19), were selected for subsequent characterization. Indeed, both huC9Mab-16 VL2 and huC9Mab-19 VL2 and VL1 show strong binding properties to the human CCR9 extracellular N-terminal region. huC9Mab-16 VL2 showed a superior binding to the other anti-CCR9 antibodies, with a significantly lower EC50.

### Cell-binding analysis of the humanized antibodies using MOLT-4 cells

Binding of the humanized anti-CCR9 antibodies was analyzed by flow cytometry using CCR9-expressing MOLT-4 cells. MOLT-4 is a T lymphoblast cell line commonly used in immunological research. Serial dilutions (1:4 titrated) of the different anti-CCR9 antibodies were incubated with MOLT-4 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant (Fig. 5).

Concentration-dependent binding of anti-CCR9 antibodies to MOLT-4 cells was observed. huC9Mab-16-VL2 exhibited particularly strong binding capacities (potency). EC₅₀ values of the different anti-CCR9 antibodies to MOLT-4 cells are listed in Table 3.

**Table 3: EC₅₀ values of humanized anti-CCR9 antibodies determined by flow cytometry using MOLT-4 cells. Mean ± SD, n=3.**

| **Antibody** | **EC₅₀ [nM]** |
|---|---|
| **SRB1** | 0.04 ± 0.01 |
| **huC9Mab16-VL1** | 1.11 ±0.37 |
| **huC9Mab16-VL2** | 0.24 ± 0.05 |
| **huC9Mab19-VL1** | 0.58 ± 0.03 |
| **huC9Mab19-VL2** | 0.59 ± 0.18 |

HuC9Mab-16-VL2 showed slightly better maximal binding compared to SRB1 (Fig. 5). Furthermore, these results indicate that humanized antibodies comprising VL2 domains are superior to those comprising VL1 domains.

### Cell-binding analysis of the humanized antibodies using HEK293-CCR9 cells

Binding of the humanized anti-CCR9 antibodies was analyzed by flow cytometry using huCCR9-expressing HEK293 cells. Serial dilutions (1:4 titrated) of the different anti-CCR9 antibodies were incubated with HEK293-CCR9 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant (Fig. 6).

Concentration-dependent binding of all anti-CCR9 antibodies to HEK293-CCR9 cells was observed. HuC9Mab-16-VL2 showed strong binding and maximal binding superior to the other anti-CCR9 molecules (Fig. 6). EC₅₀ values of the different anti-CCR9 antibodies to HEK293-CCR9 cells are listed in Table 4.

**Table 4: EC₅₀ values of humanized anti-CCR9 antibodies determined by flow cytometry using HEK293-CCR9 cells. Mean ± SD, n=3.**

| **Antibody** | **EC₅₀ [nM]** |
|---|---|
| **SRB1** | 0.04 ± 0.03 |
| **huC9Mab16-VL1** | 5.92 ± 2.28 |
| **huC9Mab16-VL2** | 0.1 ± 0.04 |
| **huC9Mab19-VL1** | 0.41 ± 0.06 |
| **huC9Mab19-VL2** | 0.92 ± 0.35 |

### Example 3

### Analysis of CCR9-ligand CCL25

Competition between the humanized antibodies and the constitutive ligand of CCR9 (CCL25) was analyzed using MOLT-4 cells (Fig. 7). Cells were pre-incubated with 100 nM of humanized anti-CCR9 antibodies for 1 hour at 4°C. After three washing steps with PBA, 400 nM CCL25 was added. Bound antibodies were detected with PE-labeled anti-human Fc antibody (Jackson ImmunoResearch 109-115-098; 1 :500), while bound CCL25-moFc was detected with FITC-labeled anti-mouse Fc antibody (Dako F0479; 1:200). Cells were analyzed using a Miltenyi MACS-Quant.

All anti-CCR9 antibodies bound to MOLT-4 cells (Fig. 7). None of the anti-CCR9 antibodies reduced binding of CCL25 to CCR9-expressing MOLT-4 cells (Fig. 7). Thus, the humanized anti-CCR9 antibodies do not interfere with ligand binding to CCR9.

### Example 4

### Epitope characterization

Human CCR9 extracellular peptides (shown in Table 5) were dissolved in PBS and diluted to a concentration of 1 mg/mL. However, N-terminal 2, ECL1 and ECL3 could not be dissolved either in PBS containing DMSO or modifying the pH of the buffer.

**Table 5: Human CCR9 extracellular peptides.**

| **CCR9 domain** | **Name** | **Peptide sequence** |
|---|---|---|
| **N-terminal region** | N-terminal 1 | TPTDFTSPIPNMADDYGSESTSS (SEQ ID NO: 22) |
| | N-terminal 2 | SSMEDYVNFNFTDFYSEKNN (SEQ ID NO: 41) |
| | N-terminal 3 | SSMEDYVNFN (SEQ ID NO: 42) |
| | N-terminal 4 | FTDFYSEKNN (SEQ ID NO: 43) |
| | N-terminal 5 | EKNNVRQFAS (SEQ ID NO: 44) |
| | N-terminal 6 | SPIPNMADDYG (SEQ ID NO: 45) |
| **ECL1** | ECL1 | DQWKFQTF (SEQ ID NO: 46) |
| **ECL2** | ECL2-1 | QIKEESGIAISTMVYPSDEST (SEQ ID NO: 47) |
| | ECL2-2 | QIKEESGIAI (SEQ ID NO: 48) |
| | ECL2-3 | TMVYPSDEST (SEQ ID NO: 49) |
| **ECL3** | ECL3 | AMFISN (SEQ ID NO: 50) |

### Peptide inhibition using 100 µg/mL of peptide

Competition with peptides representing different regions of the N-terminal domain (N-terminal 1-6 from Table 5) of CCR9 was performed for initial epitope characterization. 1 µg/mL of antibody was pre-incubated with 100 µg/mL of each peptide for 1 hour at room temperature. As a control, 1 µg/mL of the antibody was incubated without peptide. After 1 hour, the pre-incubation was transferred to MOLT-4 cells and incubated for 1 hour at 4°C. Afterwards, the cells were washed with PBA. Bound antibodies were detected with a PE-labeled anti-human Fc antibody (Jackson ImmunoResearch 109-115-098; 1:500) and cells were analyzed using a Miltenyi MACS-Quant.

The binding of the anti-CCR9 antibodies was impeded by peptide N-terminal 1 and N-terminal 6 (Fig. 8). Both N-terminal peptides comprise the epitope (PNMADD (SEQ ID NO: 23)) of SRB1. N-terminal 3, 4, and 5 did not show any impact on the binding property of the humanized anti-CCR9 antibodies. Thus, all antibodies are targeting an epitope in N-terminal 6.

### Epitope mapping of C9Mab-16 and C9Mab-19 with alanine-substituted hCCR9 peptides

To further characterize the binding epitope of C9Mab-16 and C9Mab-19, epitope mapping was performed using a 2 x Ala-scan method: 18 hCCR9 peptides, substituting two amino acid sequences with two alanine or glycine residues (Table 6).

After direct mutation of the N-terminal amino acid sequence of CCR9, C9Mab-16 does not exhibit reactions with P11A-N12A, N12A-M13A, M13A-A14G, D15A-D16A, and D16A-Y17A (Fig. 9A), and shows lower reactivity with 11OA-P11A This indicates that Pro11, Asn12, Met13, Asp15 and Asp16 are included in the critical epitope of C9Mab-16, whilst Ile10 plays an important role on antibody binding.

On the other hand, C9Mab-19 does not react with P11A-N12A, N12A-M13A, M13A-A14G, D15A-D16A, and D16A-Y17A (Fig. 9B), and showed lower reactivity with I10A-P11A and Y17A-G18A, indicating that Pro11, Asn12, Met13, Asp 15, Asp16 are included in the critical epitope of C9Mab-19, while Ile10 and Tyr17 are also involved in antibody binding.

Altogether, these results highlight the differences on the epitope showed by C9Mab-16 and C9Mab-19 as compared to 92R (murine version of SRB1) and C9Mab-1 (Takei et al. 2021, Somovilla-Crespo et al. 2018). The Ala14 residue has been considered as a key amino acid for the binding of prior art including SRB1 and C9Mab1 whilst here we demonstrate that the Ala14 residue does not participate in the antibody binding of C9Mab-16 nor of C9Mab-19. Furthermore, Ile10 seems to be involved in the epitope of C9Mab-16 and C9Mab-19 but does not appear to play a role in the epitope of SRB1.

**Table 6. The 2×alanine-substituted hCCR9 peptides.**

| **Peptides** | **Sequences** |
|---|---|
| **WT** | TDFTSPIPNMADDYGSEST (SEQ ID NO: 51) |
| **T4A-D5A** | AAFTSPIPNMADDYGSEST (SEQ ID NO: 52) |
| **D5A-F6A** | TAATSPIPNMADDYGSEST (SEQ ID NO: 53) |
| **F6A-T7A** | TDAASPIPNMADDYGSEST (SEQ ID NO: 54) |
| **T7A-S8A** | TDFAAPIPNMADDYGSEST (SEQ ID NO: 55) |
| **S8A-P9A** | TDFTAAIPNMADDYGSEST (SEQ ID NO: 56) |
| **P9A-I10A** | TDFTSAAPNMADDYGSEST (SEQ ID NO: 57) |
| **I10A-P11A** | TDFTSPAANMADDYGSEST (SEQ ID NO: 58) |
| **P11A-N12A** | TDFTSPIAAMADDYGSEST (SEQ ID NO: 59) |
| **N12A-M13A** | TDFTSPIPAAADDYGSEST (SEQ ID NO: 60) |
| **M13A-A14G** | TDFTSPIPNAGDDYGSEST (SEQ ID NO: 61) |
| **A14G-D15A** | TDFTSPIPNMGADYGSEST (SEQ ID NO: 62) |
| **D15A-D16A** | TDFTSPIPNMAAAYGSEST (SEQ ID NO: 63) |
| **D16A-Y17A** | TDFTSPIPNMADAAGSEST (SEQ ID NO: 64) |
| **Y17A-G18A** | TDFTSPIPNMADDAASEST (SEQ ID NO: 65) |
| **G18A-S19A** | TDFTSPIPNMADDYAAEST (SEQ ID NO: 66) |
| **S19A-E20A** | TDFTSPIPNMADDYGAAST (SEQ ID NO: 67) |
| **E20A-S21A** | TDFTSPIPNMADDYGSAAT (SEQ ID NO: 68) |
| **S21A-T22A** | TDFTSPIPNMADDYGSEAA (SEQ ID NO: 69) |

### Example 6

### Internalization of humanized anti-CCR9 antibodies

### pHrodo-labeling of humanized anti-CCR9 antibodies

Humanized anti-CCR9 antibodies were labeled with fluorophore (pHrodo, Sigma P36600). 300 µg of antibody was dialyzed against 0.1 M NaHCOs (pH 8.4) at 4°C overnight. The following day, the antibodies were incubated with a 20-fold excess of pHrodo fluorophore for 1 hour at room temperature in the dark. Finally, free fluorophore was removed via dialysis against 1x PBS overnight. Proteins were analyzed via SDS-PAGE and using flow cytometry. The labeling process is known to have a certain impact on the binding capacity of antibodies. Therefore, after labeling the antibodies, the binding activity of every single batch of antibodies was confirmed by flow cytometry using CCR9-expressing MOLT-4 cells. This was achieved by incubating serial dilutions (1:4 titrated) of the pHrodo-labeled anti-CCR9 antibodies with MOLT-4 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant. Concentration-dependent binding of the anti-CCR9 antibodies to MOLT-4 cells was observed in all the experiments.

### Internalization of pHrodo-labeled anti-CCR9 antibodies

The internalization of pHrodo-labeled antibodies into MOLT-4 cells was analyzed via flow cytometry, as pHrodo only shows fluorescence in acidic milieu, e.g., in the lysosome for internalized antibodies. MOLT-4 cells were seeded (20,000 cells per well) and cultivated overnight at 37 °C. The following day, 100 nM pHrodo-labeled antibody was added and incubated either at 4°C or 37°C. After 5 minutes, 60 minutes, 6 hours, 24 hours, 48 hours, and 72 hours of incubation, cells were analyzed via flow cytometry using a Miltenyi MACS-Quant. In addition, a non-binding antibody was included in this assay (RBD-specific antibody CB6).

Binding of the pHrodo-labeled antibodies was analyzed by flow cytometry using CCR9-expressing MOLT-4 cells. In this experiment, serial dilutions (1:4 titrated) of the pHrodo-labeled anti-CCR9 antibodies were incubated with cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant (Fig. 10). Concentration-dependent binding of the anti-CCR9 antibodies to MOLT-4 cells was observed. Binding of pHrodo-labeled antibodies was reduced about 1- to 4-fold. Nevertheless, SRB1 still showed the strongest binding to MOLT-4 cells followed by huC9Mab16-VL2.

**Table 7: EC₅₀ values of pHrodo-labeled anti-CCR9 antibodies determined by flow cytometry using MOLT-4 cells. n=1.**

| **Antibody** | **EC₅₀ [nM]** |
|---|---|
| **SRB1** | 0.07 |
| **huC9Mab16-VL2** | 0.27 |
| **huC9Mab19-VL2** | 0.60 |
| **SRB1 pHrodo** | 0.08 |
| **huC9Mab16-VL2 pHrodo** | 0.51 |
| **huC9Mab19-VL2 pHrodo** | 2.05 |

The internalization of pHrodo-labeled antibodies into MOLT-4 cells was analyzed via flow cytometry. All anti-CCR9 antibodies showed effective and specific internalization, compared to CB6, which is the receptor binding domain of the human anti-SARS-CoV-2 spike protein, employed as a control of unspecific internalization which does not bind CCR9.

SRB1 exhibited the strongest internalization at 10 nM (Fig. 11). HuC9Mab16-VL2 showed similar internalization to SRB1 but better internalization than huC9Mab19-VL2.

The internalization of pHrodo-labeled antibodies into MOLT-4 cells was also analyzed regarding the times at which the internalization proportion is maximal. Normalization of the results showed that the peak of internalization is reached at 24 hours for all three antibodies (SRB1, huC9Mab-16-VL2 and huC9Mab-19-VL2), and kinetic behaviour is similar for all molecules.

### Example 7

### Stability assay under thermal stress

### Freeze-thaw analysis

Samples of anti-CCR9 antibodies were frozen at -80°C for up to one day and thawed on ice. A part of the sample was used for HPLC-SEC analysis (Waters 2695 Separations Module), while the rest was frozen again at -80°C. The process was repeated for 4 times without performing in between HPLC-SEC analysis. Only after 4 days, the thawed antibodies were analyzed using SEC-HPLC.

SRB1, - huC9Mab16-VL1, and huC9Mab19-VL2 displayed one major peak proving stability during freeze-thaw-cycles (Fig. 12). HuC9Mab16-VL2 obtained a high molecular weight of 5.6%, which might be attributed to formation of multimers. However, the corresponding control was missing for the timepoint before the first freeze-thaw-cycle was started. Thus, the aggregate may have already been present in the protein solution. HuC9Mab19-VL1 showed a low molecular weight of 30.81%, which might represent degradation products.

### High temperature analysis

The anti-CCR9 antibodies were diluted to 1 mg/mL and incubated at 23°C and 40°C for 4 days. As a control, diluted antibodies were also kept at 4°C for 4 days. The stability of these antibodies was analyzed via flow cytometry analysis using CCR9-expressing MOLT-4 cells. Serial dilutions (1:10 titrated) of the anti-CCR9 antibodies at 4°C, 23°C and 40°C were incubated with MOLT-4 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant.

Binding of SRB1 to MOLT-4 cells was not affected upon incubation at higher temperatures (Fig. 13). Similarly, huC9Mab16-VL1, huC9Mab16-VL2, huC9Mab19-VL1, and huC9Mab19-VL2 showed no variations in binding under elevated temperatures.

**Table 8: EC₅₀ values of humanized anti-CCR9 antibodies after being incubated at 23°C and 40°C for 4 days. Antibodies kept at 4°C were used as a control.**

| **antibody** | **EC50 [nM]** | | |
|---|---|---|---|
| | **4°C** | **23°C** | **40°C** |
| **SRB1** | **0.05** | **0.02** | **0.05** |
| **huC9Mab-16-VL1** | **7.31** | **3.0** | **5.15** |
| **huC9Mab-16-VL2** | **0.40** | **0.40** | **0.25** |
| **huC9Mab-19 VL1** | **1.22** | **1.18** | **1.16** |
| **huC9Mab-19 VL2** | **1.86** | **2.04** | **2.00** |

### Dynamic light scattering (DLS)

The melting (aggregation) point of antibodies was determined by measuring the thermal denaturation using dynamic light scattering (DLS) with ZetaSizer Nano ZS (Malvern). 1 mg/mL antibody was diluted in 1x PBS to a total volume of 1 mL. Dynamic laser light scattering (mean count rate) was measured while the temperature was increased in 1°C intervals from 35°C to 80°C with 2 minutes equilibration time for each temperature step. The aggregation point of the antibodies was defined as the temperature at which the mean count rate was increased.

The melting point of SRB1 was determined at 64°C (Fig. 14). HuC9Mab16-VL1, huC9Mab16-VL2, huC9Mab19-VL1, and huC9Mab19-VL2 achieved similar aggregation points between 61°C and 67°C.

### Example 8

### Stability assay under pH stress

The pH stability of the anti-CCR9 antibodies was analyzed at pH 5 and pH 8.5. The antibodies were dialyzed overnight in 100 mM sodium acetate (with 150 mM NaCl) at pH 5 and in 100 mM Tris-HCl (with 150 mM NaCl) at pH 8.5. The following day, the concentration of the antibodies was measured, and the antibodies were incubated for 7 days at 40°C. After 7 days, binding of the antibody samples was analyzed via flow cytometry analysis using CCR9-expressing MOLT-4 cells. No centrifugation step was applied. Serial dilutions (1:10 titrated) of the anti-CCR9 antibodies at pH 5 or pH 8.5 were incubated with MOLT-4 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant.

Binding of the VL1 versions of the anti-CCR9 antibodies was affected at pH 5 as well as at pH 8.5 (Fig. 15). Notably, the efficacy of these antibodies was reduced. SRB1 showed similar binding at pH 8.5 as its control, while pH 5 resulted in reduced potency. In contrast, huC9Mab16-VL2 and huC9Mab19-VL2 remained unaffected by the altered pH, and did not show reduced binding at either pH 5 or pH8.

### Example 9

### In vitro serum stability

Anti-CCR9 antibodies were diluted to a final concentration of 100 nM in 50% human plasma. The samples were either directly stored at -20°C (day 0) or incubated at 37°C for 1 day, 3 days, and 7 days prior to storage at -20°C. Then, stability in human plasma was determined via flow cytometry analysis using CCR9-expressing MOLT-4 cells. Serial dilutions (1:10 titrated) of the serum-exposed anti-CCR9 antibodies were incubated with MOLT-4 cells for 1 hour at 4°C. Bound antibodies were detected with a PE-labeled anti-human Fc antibody (Jackson ImmunoResearch 109-115-098; 1:500) and cells were analyzed using a Miltenyi MACS-Quant.

SRB1 did not show any differences between the incubation periods, suggesting stability of SRB1 upon human serum incubation (Fig. 16). Similarly, huC9Mab16-VL2 displayed similar binding behavior between the incubation periods with human serum. The potency seemed to be reduced after more than 1 day of incubation for huC9Mab19-VL2. Also, the efficacy of huC9Mab16-VL1 was already decreased after 1 day of incubation with human serum. After 3 days of incubation with human serum, the efficacy of huC9Mab19-VL1 was impeded. Thus, SRB1, and huC9Mab16-VL2 seem to be stable over 7 days of human serum incubation.

### Example 10

### ADCC analysis

MOLT-4 cells were used for the ADCC analysis of the anti-CCR9 antibodies. The MOLT-4 cells were firstly stained with CFSE (CellTrace^{™} CFSE Cell Proliferation Kit; Thermo Fischer) and then 2 × 10⁴ cells were seeded to grow overnight. In addition, PBMCs from two donors (HN#1 (Fig. 17A), HN#2 (Fig. 17B)) were thawed and cultivated overnight. The following day, the PBMCs were stained with different antibodies to determine the proportion of NK cells. Cells were incubated with anti-huCD3-PE, anti-huCD56-APC and the isotype controls anti-mouselgG1-APC and anti-mouseIgG2-PE. Fluorescence signals were detected using the MACSQuant^{®} Analyzer and the proportion of NK cells was determined (HN#1: 12%; HN#2: 3.6%).

The CFSE-stained MOLT-4 cells were incubated for 15 min with a titration (1:10) of the anti-CCR9 antibodies. Then, PBMCs were added on top to reach a concentration of 2 × 10⁴ NK cells per well (equals target-to-effector ratio 1 :1). Control samples included wells with the highest concentration of antibodies but no PBMCs, wells with PBMCs only and wells including neither antibodies nor PBMCs. After incubating the plates for 24 hours, the cells were washed and resuspended in PBA containing PI. After 10 min of incubation in the dark, the viability of the CFSE-labeled MOLT-4 cells was determined by flow cytometry. For representation of data as specific lysis, all values were normalized to the control wells containing CFSE-stained MOLT-4 and PBMCs.

All anti-CCR9 antibodies mediated specific lysis of MOLT4 cells in a concentration-dependent manner (Fig. 17). HuC9Mab19-VL2 showed highest cytotoxicity at 10 nM followed by SRB1, huC9Mab16-VL2 and huC9Mab19-VL1. SRB1 and huC9Mab16-VL2 reached the best cytotoxicity at lower concentrations.

**Table 9: EC₅₀ values of humanized anti-CCR9 antibodies representing the cytotoxicity determined by flow cytometry using CFSE-stained MOLT-4 cells as well as two different PBMC batches.**

| **Antibody** | **EC₅₀ [nM]** | |
|---|---|---|
| | **HN#1** | **HN#2** |
| **SRB1** | 0.0008 | 0.003 |
| **huC9Mab16-VL1** | 0.33 | 0.44 |
| **huC9Mab16-VL2** | 0.005 | 0.01 |
| **huC9Mab19-VL1** | 0.71 | 0.38 |
| **huC9Mab19-VL2** | 0.23 | 0.06 |

### References

A number of publications are cited above in order to more fully describe and disclose the invention and the state of the art to which the invention pertains. Full citations for these references are provided below. The entirety of each of these references is incorporated herein.

Abhinandan KR, Martin AC. Analyzing the "degree of humanness" of antibody sequences. J Mol Biol. 2007 Jun 8;369(3):852-62. doi: 10.1016/j.jmb.2007.02.100. Epub 2007 Mar 14. PMID: 17442342.

Bachelerie, F., A. Ben-Baruch, A. M. Burkhardt, C. Combadiere, J. M. Farber, G. J. Graham, R. Horuk, A. H. Sparre-Ulrich, M. Locati, A. D. Luster, A. Mantovani, K. Matsushima, P. M. Murphy, R. Nibbs, H. Nomiyama, C. A. Power, A. E. Proudfoot, M. M. Rosenkilde, A. Rot, S. Sozzani, M. Thelen, O. Yoshie and A. Zlotnik (2014). "International Union of Basic and Clinical Pharmacology. [corrected]. LXXXIX. Update on the extended family of chemokine receptors and introducing a new nomenclature for atypical chemokine receptors." Pharmacol Rev 66(1): 1-79.

Carramolino, L., A. Zaballos, L. Kremer, R. Villares, P. Martin, C. Ardavín, A. C. Martinez and G. Márquez (2001). "Expression of CCR9 beta-chemokine receptor is modulated in thymocyte differentiation and is selectively maintained in CD8(+) T cells from secondary lymphoid organs." Blood 97(4): 850-857.

DeVries, M. E., A. A. Kelvin, L. Xu, L. Ran, J. Robinson and D. J. Kelvin (2006). "Defining the origins and evolution of the chemokine/chemokine receptor system." J Immunol 176(1): 401-415.

Hu, Y., L. Zhang, R. Wu, R. Han, Y. Jia, Z. Jiang, M. Cheng, J. Gan, X. Tao and Q. Zhang (2011). "Specific killing of CCR9 high-expressing acute T lymphocytic leukemia cells by CCL25 fused with PE38 toxin." Leuk Res 35(9): 1254-1260.

Johnson-Holiday, C., R. Singh, E. Johnson, S. Singh, C. R. Stockard, W. E. Grizzle and J. W. Lillard, Jr. (2011). "CCL25 mediates migration, invasion and matrix metalloproteinase expression by breast cancer cells in a CCR9-dependent fashion." Int J Oncol 38(5): 1279-1285.

Kunkel, E. J., J. J. Campbell, G. Haraldsen, J. Pan, J. Boisvert, A. I. Roberts, E. C. Ebert, M. A. Vierra, S. B. Goodman, M. C. Genovese, A. J. Wardlaw, H. B. Greenberg, C. M. Parker, E. C. Butcher, D. P. Andrew and W. W. Agace (2000). "Lymphocyte CC chemokine receptor 9 and epithelial thymus-expressed chemokine (TECK) expression distinguish the small intestinal immune compartment: Epithelial expression of tissue-specific chemokines as an organizing principle in regional immunity." J Exp Med 192(5): 761-768.

Lazennec, G. and A. Richmond (2010). "Chemokines and chemokine receptors: new insights into cancer-related inflammation." Trends Mol Med 16(3): 133-144.

Mirandola, L., M. Chiriva-Internati, D. Montagna, F. Locatelli, M. Zecca, M. Ranzani, A. Basile, M. Locati, E. Cobos, W. M. Kast, R. Asselta, E. M. Paraboschi, P. Comi and R. Chiaramonte (2012). "Notch1 regulates chemotaxis and proliferation by controlling the CC-chemokine receptors 5 and 9 in T cell acute lymphoblastic leukaemia." J Pathol 226(5): 713-722.

Mukaida, N. and T. Baba (2012). "Chemokines in tumor development and progression." Exp Cell Res 318(2): 95-102.

Pathak, M; Lal, G. 2020. The Regulatory Function of CCR9(+) Dendritic Cells in Inflammation and Autoimmunity. Front. Immunol. 11. 1-8.

Qiuping, Z., et al., Selectively increased expression and functions of chemokine receptor CCR9 on CD4+ T cells from patients with T-cell lineage acute lymphocytic leukemia. Cancer Res, 2003. 63(19): p. 6469-77.

Raman, D., T. Sobolik-Delmaire and A. Richmond (2011). "Chemokines in health and disease." Exp Cell Res 317(5): 575-589.

Sarvaiya, P. J., D. Guo, I. Ulasov, P. Gabikian and M. S. Lesniak (2013). "Chemokines in tumor progression and metastasis." Oncotarget 4(12): 2171-2185.

Sharma, P. K., R. Singh, K. R. Novakovic, J. W. Eaton, W. E. Grizzle and S. Singh (2010). "CCR9 mediates PI3K/AKT-dependent antiapoptotic signals in prostate cancer cells and inhibition of CCR9-CCL25 interaction enhances the cytotoxic effects of etoposide." Int J Cancer 127(9): 2020-2030.

Takei, J., et al., Epitope Mapping of an Anti-Human CCR9 Monoclonal Antibody (C9Mab-1) Using Enzyme-Linked Immunosorbent Assay. Monoclon Antib Immunodiagn Immunother, 2021. 40(6): p. 239-242.

Somovilla-Crespo, B., et al., 92R Monoclonal Antibody Inhibits Human CCR9(+) Leukemia Cells Growth in NSG Mice Xenografts. Front Immunol, 2018. 9: p. 77.

Trivedi, P. J., & Adams, D. H. (2018). Chemokines and Chemokine Receptors as Therapeutic Targets in Inflammatory Bowel Disease; Pitfalls and Promise. Journal of Crohn's & colitis, 12(suppl_2), S641-S652. https://doi.org/10.1093/ecco-jcc/jjx145

Tu, Z., R. Xiao, J. Xiong, K. M. Tembo, X. Deng, M. Xiong, P. Liu, M. Wang and Q. Zhang (2016). "CCR9 in cancer: oncogenic role and therapeutic targeting." J Hematol Oncol 9: 10.

Weitzenfeld, P. and A. Ben-Baruch (2014). "The chemokine system, and its CCR5 and CXCR4 receptors, as potential targets for personalized therapy in cancer." Cancer Lett 352(1): 36-53.

Wendland, M., N. Czeloth, N. Mach, B. Malissen, E. Kremmer, O. Pabst and R. Förster (2007). "CCR9 is a homing receptor for plasmacytoid dendritic cells to the small intestine." Proc Natl Acad Sci U S A 104(15): 6347-6352.

Wu, W., et al., Strong expression of chemokine receptor CCR9 in diffuse large B-cell lymphoma and follicular lymphoma strongly correlates with gastrointestinal involvement. Hum Pathol, 2014. 45(7): p. 1451-8.

Wu, X., Sun, M., Yang, Z., Lu, C., Wang, Q., Wang, H., Deng, C., Liu, Y., & Yang, Y. (2021). The Roles of CCR9/CCL25 in Inflammation and Inflammation-Associated Diseases. Frontiers in cell and developmental biology, 9, 686548.

Wurbel, M. A., B. Malissen and J. J. Campbell (2006). "Complex regulation of CCR9 at multiple discrete stages of T cell development." Eur J Immunol 36(1): 73-81.

Youn, B. S., C. H. Kim, F. O. Smith and H. E. Broxmeyer (1999). "TECK, an efficacious chemoattractant for human thymocytes, uses GPR-9-6/CCR9 as a specific receptor." Blood 94(7): 2533-2536.

Zaballos, A., J. Gutiérrez, R. Varona, C. Ardavín and G. Márquez (1999). "Cutting edge: identification of the orphan chemokine receptor GPR-9-6 as CCR9, the receptor for the chemokine TECK." J Immunol 162(10): 5671-5675.

Zabel, B. A., W. W. Agace, J. J. Campbell, H. M. Heath, D. Parent, A. I. Roberts, E. C. Ebert, N. Kassam, S. Qin, M. Zovko, G. J. LaRosa, L. L. Yang, D. Soler, E. C. Butcher, P. D. Ponath, C. M. Parker and D. P. Andrew (1999). "Human G protein-coupled receptor GPR-9-6/CC chemokine receptor 9 is selectively expressed on intestinal homing T lymphocytes, mucosal lymphocytes, and thymocytes and is required for thymus-expressed chemokine-mediated chemotaxis." J Exp Med 190(9): 1241-1256.

Zlotnik, A. and O. Yoshie (2000). "Chemokines: a new classification system and their role in immunity." Immunity 12(2): 121-127.

### SEQUENCES

*SEQ ID NO: 1* - *Variable Light Chain (16)* 
DVVMTQSPSSLSASVGDRVTITCRSSQSLVHSNGITFLHWYQQKPGKAPKLLIYKVSNRFSGVPSRFSGS
GSGTDFTLTISSLQPEDFATYYCSQTTHVPWTFGGGTKVEIK*
*SEQ ID NO: 2* - *CDR-L 1*
   RSSQSLXHSNGXTFLH
*SEQ ID NO: 3* - *CDR-L2*
   KVSNRFS
*SEQ ID NO: 4* - *CDR-L3*
   SQTTHXPWT
*SEQ ID NO: 5* - *CDR-L 1*
   RSSQSLVHSNGITFLH
*SEQ ID NO: 6* - *CDR-L 1*
   RSSQSLLHSNGNTFLH
*SEQ ID NO: 7 - CDR-L3*
   SQTTHVPWT
*SEQ ID NO: 8* - *CDR-L3*
   SQTTHLPWT
*SEQ ID NO: 9* - *CDR-H1*
   GFTFSXYWMX
*SEQ ID NO: 10 - CDR-H2*
   EIRSKSXNYATHYXESXXG
*SEQ ID NO: 11* - *CDR-H3*
   XYSPY
*SEQ ID NO: 12* - *CDR-H1*
   GFTFSDYWMN
*SEQ ID NO: 13* - *CDR-H1*
   GFTFSNYWMD
*SEQ ID NO: 14* - *CDR-H2*
   EIRSKSKNYATHYEESVRG
*SEQ ID NO: 15* - *CDR-H2*
   EIRSKSNNYATHYAESMKG
*SEQ ID NO: 16* - *CDR-H3*
   FYSPY
*SEQ ID NO: 17 - CDR-H3*
   YYSPY
*SEQ ID NO: 18 - Variable heavy chain (16)* 
EVQLVESGGGLVKPGGSLRLSCAASGFTFSDYWMNWVRQAPGKGLEWVGEIRSKSKNYATHYEESVR
GRFTISRDDSKNTLYLQMNSLKTEDTAVYYCMFFYSPYWGQGTLVTVSS*
*SEQ ID NO: 19 - Variable heavy chain (19)* 
EVQLVESGGGLVKPGGSLRLSCAASGFTFSNYWMDWVRQAPGKGLEWVGEIRSKSNNYATHYAESMK
GRFTISRDDSKNTLYLQMNSLKTEDTAVYYCAFYYSPYWGQGTLVTVSS*
*SEQ ID NO: 20* - *Variable light chain (19)* 
DVVMTQSPSSLSASVGDRVTITCRSSQSLLHSNGNTFLHWYQQKPGKAPKLLIYKVSNRFSGVPSRFSG
SGSGTDFTLTISSLQPEDFATYYCSQTTHLPWTFGGGTKVEIK*
*SEQ ID NO: 21* - *amino acids 2-22 of N-terminal extracellular domain of CCR9 isoform A*
   TPTDFTSPIPNMADDYGSEST
*SEQ ID NO: 22* - *amino acids 2-24 of human CCR9 extracellular N-terminal region*
   TPTDFTSPIPNMADDYGSESTSS
*SEQ ID NO: 23*
   PNMADD
*SEQ ID NO: 24*
   TSPIPNMADDYGS
*SEQ ID NO: 25*
   **A**SPIPNMADDYGS
*SEQ ID NO: 26*
   TSAIPNMADDYGS
*SEQ ID NO: 27*
   TSP**A**PNMADDYGS
*SEQ ID NO: 28*
   TSPI**A**NMADDYGS
*SEQ ID NO: 29*
   TSPIP**A**MADDYGS
*SEQ ID NO: 30*
   TSPIPN**A**ADDYGS
*SEQ ID NO: 31*
   TSPIPNM**G**DDYGS
*SEQ ID NO: 32*
   TSPIPNMA**A**DYGS
*SEQ ID NO: 33*
   TSPIPNMAD**A**YGS
*SEQ ID NO: 34*
   TSPIPNMADDAGS
*SEQ ID NO: 35*
   TSPIPNMADDY**A**S
*SEQ ID NO: 36*
   TSPIPNMADDYG**A**
*SEQ ID NO: 37*
   RSSQSLVHSNGNTYLN
*SEQ ID NO: 38*
   SQSTHFPRT
*SEQ ID NO: 39* 
DVVMTQSPSSLSASVGDRVTITC RSSQSLXHSNGXTFLH WYQQKPGKAPKLLIY KVSNRFS
GVPSRFSGSGSGTDFTLTISSLQPEDFATYYC SQTTHXPWT FGGGTKVEIK*
*SEQ ID NO: 40* 
DVVMTQSPSSLSASVGDRVTITC RSSQSLXHSNGXTFLH

WYQQKPGKAPKLLIYKVSNRFSGVPSRFSGSGSGTDFTLTISSLQPEDFATYYC SQTTHXPWT
 FGGGTKVEIK*
*SEQ ID NO: 41*
   SSMEDYVNFNFTDFYSEKNN
*SEQ ID NO: 42*
   SSMEDYVNFN
*SEQ ID NO: 43*
   FTDFYSEKNN
*SEQ ID NO: 44*
   EKNNVRQFAS
*SEQ ID NO: 45*
   SPIPNMADDYG
*SEQ ID NO: 46*
   DQWKFQTF
*SEQ ID NO: 47*
   QIKEESGIAISTMVYPSDEST
*SEQ ID NO: 48*
   QIKEESGIAI
*SEQ ID NO: 49*
   TMVYPSDEST
*SEQ ID NO: 50*
   AMFISN
*SEQ ID Nos 51 to 69* TDFTSPIPNMADDYGSEST (SEQ ID NO: 51) AAFTSPIPNMADDYGSEST (SEQ ID NO: 52) TAATSPIPNMADDYGSEST (SEQ ID NO: 53) TDAASPIPNMADDYGSEST (SEQ ID NO: 54) TDFAAPIPNMADDYGSEST (SEQ ID NO: 55) TDFTAAIPNMADDYGSEST (SEQ ID NO: 56) TDFTSAAPNMADDYGSEST (SEQ ID NO: 57) TDFTSPAANMADDYGSEST (SEQ ID NO: 58) TDFTSPIAAMADDYGSEST (SEQ ID NO: 59) TDFTSPIPAAADDYGSEST (SEQ ID NO: 60) TDFTSPIPNAGDDYGSEST (SEQ ID NO: 61) TDFTSPIPNMGADYGSEST (SEQ ID NO: 62) TDFTSPIPNMAAAYGSEST (SEQ ID NO: 63) TDFTSPIPNMADAAGSEST (SEQ ID NO: 64) TDFTSPIPNMADDAASEST (SEQ ID NO: 65) TDFTSPIPNMADDYAAEST (SEQ ID NO: 66) TDFTSPIPNMADDYGAAST (SEQ ID NO: 67) TDFTSPIPNMADDYGSAAT (SEQ ID NO: 68) TDFTSPIPNMADDYGSEAA (SEQ ID NO: 69)
*SEQ ID NO: 70 (SR81-VH* *Figure 1**)* 
EVQLVESGGGLVKPGGSLRLSCAAS GFTFSKFWMN WVRQAPGKGLEWVG
EIRLKSNNYATHYAESVKG RFTISRDDSKNTLYLQMNSLKTEDTAVYYCTS DGWFAY
WGQGTLVTVSS
*SEQ ID NO: 71 (SRB10-VH* *Figure 1**)* 
EVKLEESGGGLVQPGGSMKLSCVAS GFTFNKFWMN WVRQSPEKGLEWVA
EIRLKSNNYATHYAESVKG RFTISRDDSKSSVYLQMNNLRAEDTGIYYCAS DGWFAY
WGQGTLVTVSS
*SEQ ID NO: 72 (IAb01-VH* *Figure 1**)* 
EVKVEESGGGLVQPGGSMKLSCVVS GLTFSNFWMT WVRQSPEKGLEWVA
EIRLKSNNYATHYAESVKG RFTISRDDSRSSVFLQMNNLRAEDTGIYYCTT DGWFAF
WGQGTLVTVSA
*SEQ ID NO: 73 (IAb02-VH* *Figure 1**)* 
EVKVEESGGGLVQPGGSMKLSCAAS GFIFSNFWMN WVRQSPEKGLEWIA
EIRLKSNNYATHYAESVKG RFTISRDDSKSSVYLQMNNLRAEDTGIYYCAT DGWFAF
WGQGTLVTVSA
*SEQ ID NO: 74 (C9M16-VH* *Figure 1**)* 
EVKLEESGGGLVLPGGSMKLSCVAS GFTFSDYWMN WVRQSPEKGLEWVA
EIRSKSKNYATHYEESVRG RCTISRDDSKSSVYLQMNNLRAEDTGIYFCMF FYS-PY
WGQGTLVTVSA
*SEQ ID NO: 75 (C9M19-VH* *Figure 1**)* 
EVKLEESGGGLVQPGGSMKLSCVAS GFTFSNYWMD WVRQSPEKGLEWVA
EIRSKSNNYATHYAESMKG RFTISRDDSKSSVYLQMINLRTEDTGIYYCAF YYS-PY
WGQGTLVTVSA
*SEQ ID NO: 76 (SRB1-VL* *Figure 1**)* 
DVQMTQSPSSLSASVGDRVTITC RSSQSLVHSNGNTYLN WYQQKPGKAPKLLIY KVSNRFS
GVPSRFSGSGSGTDFTLTISSLQPEDFATYYC SQSTHFPRT FGGGTKVEIKR
*SEQ ID NO: 77 (SRB 10- VL* *Figure 1**)* 
DVVMTQTPLSLPVSLGDQASISC RSSQSLVHSNGNTYLN WCLQRPGQSPKSLIY KVSNRFS
GVPDRFSGSGSGTDFTLKISRVEAEDLGVYFC SQSTHFPRT FGGGTKLEIKR
*SEQ ID NO: 78 (IAb01-VL* *Figure 1**)* 
QIVLTQSPTIMSASPGEKVTITC SASSSVSY------MH WFQQKPGTSPKLWIY STSNLAS
GVPARFSGSGSGTSYSLTISRMEAEDAATYYC QQRSSYPFT FGSGTKLEIKR
*SEQ ID NO: 79 (IAb02-VL* *Figure 1**)* 
DVVMTQTPLSLPVSLGDQASISC RSSQSLVHSNGNTYLQ WHLQKPGQSPKFLIY KVSNRFS
GVPDRFSGSGSGTDFTLKISRVEAEDLGVYFC SQSTHVPRT FGGGTKLEIKR
*SEQ ID NO: 80 (C9M16-VL* *Figure 1**)* 
DVVMTQTPLSLPVSLGDQASISC RSSQSLVHSNGITFLH WYLQKPGQSPKLLIY KVSNRFS
GVPDRFSGSGSGTDFTLKISRVEAEDLGVYFC SQTTHVPWT FGGGTKLEIKR
*SEQ ID NO: 81 (C9M19-VI* *Figure 1**)* 
DVVMTQTPLSLPVSLGDQASISC RSSQSLLHSNGNTFLH WYLQKPGQSPKLLIY KVSNRFS
GVPDRFSGSGSGTDFTLKVSRVEAEDLGVYFC SQTTHLPWT FGGGTKLEIKR
*SEQ ID NO: 82*
   XFXYSPY
*SEQ ID NO: 83*
   MFXYSPY
*SEQ ID NO: 84*
   AFXYSPY
*SEQ ID NO: 85*
   MFFYSPY
*SEQ ID NO: 86*
   AFYYSPY

## Claims

1. An anti-CCR9 antibody or antigen binding fragment thereof, comprising a variable light chain comprising SEQ ID NO:1 or a variant thereof having at least 95% sequence identity to SEQ ID NO:1, wherein the variable light chain comprises a CDR-L1 comprising the amino acid sequence RSSQSLXHSNGXTFLH (SEQ ID NO: 2), a CDR-L2 comprising the amino acid sequence KVSNRFS (SEQ ID NO: 3) and a CDR-L3 comprising the amino acid sequence SQTTHXPWT (SEQ ID NO: 4), wherein X can be any amino acid.

2. The anti-CCR9 antibody or antigen binding fragment thereof of claim 1, wherein the CDR-L1 comprises the amino acid sequence RSSQSLVHSNGITFLH (SEQ ID NO: 5) or the amino acid sequence RSSQSLLHSNGNTFLH (SEQ ID NO: 6).

3. The anti-CCR9 antibody or antigen binding fragment thereof of claim 1 or claim 2, wherein the CDR-L3 comprises the amino acid sequence SQTTHVPWT (SEQ ID NO: 7) or the amino acid sequence SQTTHLPWT (SEQ ID NO: 8).

4. The anti-CCR9 antibody or antigen binding fragment thereof of any one of the preceding claims,
wherein the antibody or antigen binding fragment thereof comprises a CDR-H1 comprising the amino acid sequence GFTFSXYWMX (SEQ ID NO: 9), a CDR-H2 comprising the amino acid sequence EIRSKSXNYATHYXESXXG (SEQ ID NO: 10) and a CDR-H3 comprising the amino acid sequence XYSPY (SEQ ID NO: 11), wherein X can be any amino acid.

5. The anti-CCR9 antibody or antigen binding fragment thereof of claim 4, wherein the CDR-H1 comprises the amino acid sequence GFTFSDYWMN (SEQ ID NO: 12) or the amino acid sequence GFTFSNYWMD (SEQ ID NO: 13).

6. The anti-CCR9 antibody or antigen binding fragment thereof of claim 4 or claim 5, wherein the CDR-H2 comprises the amino acid sequence EIRSKSKNYATHYEESVRG (SEQ ID NO: 14) or the amino acid sequence EIRSKSNNYATHYAESMKG (SEQ ID NO: 15).

7. The anti-CCR9 antibody or antigen binding fragment thereof of any one of claims 4 to 6, wherein the CDR-H3 comprises the amino acid sequence FYSPY (SEQ ID NO: 16) or the amino acid sequence YYSPY (SEQ ID NO: 17).

8. The anti-CCR9 antibody or antigen binding fragment thereof of any one of the preceding claims,
wherein the antibody or antigen binding fragment thereof comprises a variable heavy chain comprising SEQ ID NO: 18 or a variant thereof having at least 95% sequence identity to SEQ ID NO: 18.

9. The anti-CCR9 antibody or antigen binding fragment thereof of any one of the preceding claims, wherein the antibody or antigen binding fragment thereof comprises a variable heavy chain comprising SEQ ID NO: 18 or SEQ ID NO: 19.

10. The anti-CCR9 antibody or antigen binding fragment thereof of any one of the preceding claims, wherein the variable light chain comprises SEQ ID NO: 1 or SEQ ID NO: 20.

11. The anti-CCR9 antibody or antigen binding fragment thereof of any one of the preceding claims, wherein the antibody or antigen binding fragment thereof comprises:
a. A variable light chain comprising SEQ ID NO:1, wherein the variable light chain comprises a CDR-L1 comprising the amino acid sequence RSSQSLVHSNGITFLH (SEQ ID NO: 5), a CDR-L2 comprising the amino acid sequence KVSNRFS (SEQ ID NO: 3), and a CDR-L3 comprising the amino acid sequence SQTTHVPWT (SEQ ID NO: 7); and
b. A variable heavy chain comprising a CDR-H1 comprising the amino acid sequence GFTFSDYWMN (SEQ ID NO: 12), a CDR-H2 comprising the amino acid sequence EIRSKSKNYATHYEESVRG (SEQ ID NO: 14) and a CDR-H3 comprising the amino acid sequence FYSPY (SEQ ID NO: 16).

12. The anti-CCR9 antibody or antigen binding fragment thereof of any one of the preceding claims, wherein the antibody or antigen binding fragment thereof binds to an epitope comprising amino acids at positions 11, 12, 13, 15 and 16 of the N-terminal extracellular domain of CCR9 isoform A.

13. The anti-CCR9 antibody or antigen binding fragment thereof of any one of the preceding claims, wherein the anti-CCR9 antibody or antigen binding fragment thereof comprises an IgG anti-CCR9 antibody.

14. The anti-CCR9 antibody or antigen binding fragment thereof of any one of the preceding claims, wherein the antibody or antigen binding fragment thereof exhibits a binding affinity dissociation constant K_{D} for human CCR9 of about 1 x 10⁻⁶ M or less.

15. An antibody drug conjugate (ADC) comprising the anti-CCR9 antibody or antigen binding fragment thereof of any one of the preceding claims conjugated to a drug, optionally a cytotoxic or chemotherapy drug.

16. A polynucleotide comprising a nucleotide sequence encoding the anti-CCR9 antibody or antigen binding fragment thereof of any one of claims 1 to 14.

17. A vector comprising the polynucleotide of claim 16.

18. A cell comprising the anti-CCR9 antibody or antigen binding fragment thereof of any one of claims 1 to 14, the polynucleotide of claim 16 or the vector of claim 17.

19. A pharmaceutical composition comprising the anti-CCR9 antibody or antigen binding fragment thereof of any one of claims 1 to 14, the ADC of claim 15, the polynucleotide of claim 16, the vector of claim 17 or the cell of claim 18 and a pharmaceutically or physiologically acceptable diluent and/or carrier.

20. A kit comprising the anti-CCR9 antibody or antigen binding fragment thereof of any one of claims 1 to 14, the ADC of claim 15, the polynucleotide of claim 16, the vector of claim 17, the cell of claim 18 or the pharmaceutical composition of claim 19.

21. A method of diagnosing a disease associated with elevated CCR9 expression in a subject, the method comprising:
a) contacting the antibody or antigen binding fragment thereof of any one of claims 1 to 14 with a sample comprising cells from the subject;
b) detecting and/or quantifying CCR9 expression in the subject sample;
c) comparing the level of CCR9 expression detected in the subject sample with the level of CCR9 expression detected in a control sample;
d) correlating the comparison obtained in step c) with the presence of a disease associated with elevated CCR9 expression.

22. The anti-CCR9 antibody or antigen binding fragment thereof of any one of claims 1 to 14, the ADC of claim 15, the polynucleotide of claim 16, the vector of claim 17, the cell of claim 18 or the pharmaceutical composition of claim 19 for use in the treatment or prevention of a disease.

23. The method of claim 21 or the anti-CCR9 antibody or antigen binding fragment thereof, the ADC, the polynucleotide, the vector, the cell or the pharmaceutical composition for use of claim 22, wherein the disease is an inflammatory disease.

24. The method of claim 21 or claim 23 or the anti-CCR9 antibody or antigen binding fragment thereof, the ADC, the polynucleotide, the vector, the cell or the pharmaceutical composition for use of claim 22 or claim 23, wherein the disease is selected from cancer, Crohn's disease, colitis, ulcerative colitis, inflammatory bowel disease, liver fibrosis and acute liver inflammation, optionally wherein the disease is cancer or inflammatory bowel disease, further optionally wherein the disease is inflammatory bowel disease.

25. The method of any of claims 21, 23 or 24 or the anti-CCR9 antibody or antigen binding fragment thereof, the ADC, the polynucleotide, the vector, the cell or the pharmaceutical composition for use of any of claims 22 to 24, where the disease is cancer.

26. The method of claim 24 or claim 25 or the anti-CCR9 antibody or antigen binding fragment thereof, the ADC, the polynucleotide, vector, cell or pharmaceutical composition for use of claim 24 or claim 25, wherein the cancer is selected from ovarian cancer, prostate cancer, breast cancer, melanoma, T-cell acute lymphoblastic leukaemia (T-ALL), pancreatic cancer, colorectal cancer, lung cancer, circulating cells from a solid tumour (CTCs), T-cell lineage lymphomas, acute myeloid leukaemia (AML) and lung cancer.
